# FASCICULE DE BREVET EUROPEEN

(11) **EP 2 195 420 B1**
(45) Date de publication et mention de la délivrance du brevet: **08.04.2015**
(21) Numéro de dépôt: 08840554.3
(22) Date de dépôt: 26.09.2008
(51) Int. Cl.: C12N 7/04, A61K 39/145, A61P 31/16, C12N 7/00, C12N 15/44, C12R 1/93, A61K 35/76, A61P 37/04

(54) **PROCEDE DE PRODUCTION DU VIRUS DE LA GRIPPE**
VERFAHREN ZUR HERSTELLUNG DES GRIPPEVIRUS
METHOD FOR PRODUCING FLU VIRUS

(30) Priorité: 26.09.2007 FR 0757884
(43) Date de publication de la demande: 16.06.2010
(73) Titulaire: Sanofi Pasteur, 69367 Lyon Cedex 07 (FR); Merial Limited, Duluth, GA 30096-4640 (US)
(72) Inventeur: GERDIL, Catherine, 69160 Tassin La Demi Lune (FR); MOSTE, Catherine, 69260 Charbonnières Les Bains (FR); LEGASTELOIS, Isabelle, 69700 Saint Andeol Le Château (FR); BUBLOT, Michel, 69630 Chaponost (FR); LE GROS, François-Xavier, 69230 Saint Genis Laval (FR)
(74) Mandataire: Hurpin, Christian Marcel
(86) Numéro de dépôt international: PCT/FR2008/051730
(87) Numéro de publication internationale: WO 2009/050390

(56) Documents cités:
- WO-A-01/37810
- WAREING M D ET AL: "Live attenuated vaccines against influenza; an historical review" VACCINE, BUTTERWORTH SCIENTIFIC. GUILDFORD, GB, vol. 19, no. 25-26, 14 mai 2001 (2001-05-14), pages 3320-3330, XP004238926 ISSN: 0264-410X
- BUBLOT ET AL: "Use of a Vectored Vaccine against Infectious Bursal Disease of Chickens in the Face of High-Titred Maternally Derived Antibody" JOURNAL OF COMPARATIVE PATHOLOGY, ACADEMIC PRESS, LONDON, GB, vol. 137, 28 juin 2007 (2007-06-28), pages S81-S84, XP022128831 ISSN: 0021-9975
- STONE HENRY D ET AL: "Simulation of maternal immunity by inoculation of immune yolk preparations into the yolk sac of 1-day-old chickens" AVIAN DISEASES, vol. 36, no. 4, 1992, pages 1048-1051, XP009097205 ISSN: 0005-2086 cité dans la demande
- STEENSELS M ET AL: "Efficacy of an inactivated and a fowlpox-vectored vaccine in muscovy ducks against an Asian H5N1 highly pathogenic avian influenza viral challenge" AVIAN DISEASES, vol. 51, no. 1, Suppl. S, mars 2007 (2007-03), pages 325-331, XP009097236 ISSN: 0005-2086
- BUBLOT MICHEL ET AL: "Efficacy of a fowlpox-vectored avian influenza H5 vaccine against Asian H5N1 highly pathogenic avian influenza virus challenge." AVIAN DISEASES MAR 2007, vol. 51, no. 1 Suppl, mars 2007 (2007-03), pages 498-500, XP009097245 ISSN: 0005-2086

## Description

L'invention a pour objet un procédé de production du virus de la grippe en utilisant des oeufs provenant de poules vaccinées contre la grippe ainsi que l'utilisation d'un tel procédé pour la fabrication d'un vaccin contre la grippe.

On connait actuellement trois types de virus de la grippe (A, B et C), les virus de type A étant responsables d'affections animales et humaines tandis que les virus de types B et C sont surtout pathogènes pour l'homme. Les virus de type A sont subdivisés en sous types en fonction de la structure antigénique de l'hémagglutinine (HA) et de la neuraminidase (NA) qui sont les principales glycoprotéines de l'enveloppe virale. On distingue 16 sous types d'HA (H1 à H 16) et 9 sous types de NA (N1 à N9). Le sous type d'un virus de type A est donc défini par le sous type d'HA et le sous type de NA qui sont présentes dans l'enveloppe virale. Les oiseaux sauvages constituent le réservoir de tous les sous-types d'influenza A. Certains sous-types de virus influenza de type A infectent de manière endémique ou épidémique (épidémies annuelles) les oiseaux domestiques (divers sous-types dont H5N1 et H9N2), le cheval (H3N8 principalement), le porc (H1N1, H3N2, et H1N2 principalement) ainsi que l'homme (H1N1 et H3N2 principalement). Le chien, le chat et d'autres espèces sauvages peuvent également occasionnellement être infectées par certains sous-types (H3N8 et H5N1 chez le chien ; H5N1 chez le chat).

Dans le domaine vétérinaire, les élevages de volailles, et plus particulièrement les poussins, les poulets, les poules et les coqs représentent en nombre la population la plus importante susceptible d'être affectée par le virus de la grippe. Les souches de grippe aviaire de sous-types H5 et H7 peuvent être de deux pathotypes : un pathotype faiblement pathogène (« low path » ou LP) et un pathotype hautement pathogène (« high path » ou HP). Les souches HP sont responsables de la peste aviaire et dérivent des souches LP H5 et H 7 après mutations/insertions notamment au niveau du site de clivage de l'hémagglutinine (présence de multiples acides aminés basiques). Jusqu'à présent, on préconise surtout des mesures sanitaires strictes et des contrôles réguliers dans les élevages pour prévenir la grippe aviaire, et en particulier l'infection par les sous-types H5 et H7.

Chez l'homme, on préconise la vaccination contre les souches virales circulantes saisonnières responsables d'épidémies plus ou moins importantes selon les années. La plupart des vaccins actuels sont produits en utilisant des oeufs embryonnés de poules, ces oeufs étant infectés avec trois souches de virus grippal différentes (deux souches de virus grippal de type A ayant le sous type H3N2 et H1N1 et une souche de virus de type B). On utilise des oeufs embryonnés de poules non vaccinées contre la grippe pour éviter tout phénomène d'interférence qui pourrait nuire à la réplication du virus. On sait en effet que les anticorps maternels sont transférés aux poussins après avoir séjourné dans l'oeuf et les protègent contre les infections microbiennes pendant les premiers jours de la vie mais en contre partie ils sont la cause d'une immunité déficiente si on vaccine prématurément des poussins contre un agent microbien alors qu'il existe encore des anticorps protecteurs maternels contre cet agent. Stone H. et coll. (1992, Avian Dis. 36: 1048-1051) ont montré qu'on pouvait immuniser de façon passive des poussins nouveau-nés contre la maladie de Newcastle en inoculant du jaune d'oeufs provenant de poules immunisées contre le virus de la maladie de Newcastle (NDV). Cependant, si à la suite de l'administration des jaunes d'oeufs, on vaccine les poussins avec le virus NDV, on observe une baisse de la réponse immunitaire au vaccin. On sait aussi d'après les travaux d' Hamal et coll. (2006, Poultry Science 65: 1364-1372) que les taux de transfert des anticorps maternels protecteurs aux poussins nouveau-nés, notamment le transfert des anticorps dirigés contre le virus NDV ou le virus de la bronchite infectieuse (IBV), se situent entre 30 % et 40 % (pourcentage du taux d'anticorps dans le plasma de la poule circulant dans le sang du poussin de trois jours), ce qui indique qu'une grande quantité des anticorps maternels sont séquestrés dans l'oeuf. Ceci est confirmé par les travaux de Beck J.R et coll. (2003, Avian Dis. 47:1196-1199) qui montrent que tous les oeufs contiennent des anticorps anti HA, environ 3 semaines après avoir vacciné des poules avec une souche de virus grippal inactivé. On sait enfin d'après les travaux de Fontaine et coll. (1963, Pathobiologie, 11/9: 611-613) que si on inocule du sérum antigrippal à des oeufs embryonnés, on protège les oeufs contre l'infection du virus grippal. Toutes ces raisons ont amené l'homme de l'art à considérer que si on utilisait des oeufs de poules vaccinées contre la grippe, en raison du transfert des anticorps maternels dirigés contre la grippe dans les oeufs, ceux-ci deviendraient inaptes à la production de virus grippaux.

Depuis le début des années 2000, l'incidence économique de la grippe aviaire dans les élevages d'oiseaux domestiques ne cesse de croître avec l'apparition de souches de virus aviaires hautement contagieuses et pathogènes, décimant des élevages entiers de poulets ou dans les cas moins dramatiques stoppant la ponte chez les poules infectées. Le typage des HA des souches de virus hautement pathogènes montre qu'elles ont presque toutes le sous type H5 ou H7. On craint maintenant que les souches de virus ayant le sous type H5 ou H7 ne s'adaptent à l'homme et puissent être à l'origine d'une véritable pandémie de grippe chez l'homme ; des cas sévères de grippe humaine, certes isolés, impliquant ces sous types ont déjà été rapportés.

Devant le risque que la fourniture d'oeufs puisse ne plus être toujours assurée pour fabriquer le vaccin antigrippal, les nouvelles méthodes de production de vaccins contre la grippe s'orientent actuellement vers l'utilisation de systèmes de culture cellulaire.

### Résumé de l'invention

En dépit des nouveaux modes de production des vaccins grippaux, il existe néanmoins toujours un besoin de pouvoir produire, en toutes circonstances, dans un temps réduit et en très grande quantité du virus grippal pour fabriquer le vaccin contre la grippe. La présente invention répond à ce besoin en décrivant un procédé de production du virus de la grippe basé, contre toute attente, sur l'utilisation d'oeufs provenant de poules préalablement vaccinées contre la grippe.

Un objet de l'invention concerne en effet :
Un procédé de production du virus de la grippe selon lequel :
   a. on administre à des poules un vaccin contre la grippe,
   b. on prélève les oeufs des poules vaccinées,
   c. on déclenche le processus d'embryogénèse,
   d. on infecte les oeufs embryonnés en inoculant un virus de la grippe dans la cavité allantoïque,
   e. on incube les oeufs embryonnés infectés dans des conditions de température et d'humidité qui permettent la réplication du virus et,
   f. on récolte le liquide allantoïque contenant le virus.

De façon préférée, le vaccin protège les poules contre la grippe aviaire.

Typiquement, le vaccin contre la grippe comprend dans sa composition l'hémagglutinine d'un virus de la grippe sous la forme de protéine et/ou de gène codant pour cette protéine. Selon un aspect, la composition du vaccin contre la grippe contient un virus grippal entier et inactivé.

Selon un autre aspect, la composition du vaccin contre la grippe contient un produit dérivé d'un virus grippal entier.

Selon encore un autre aspect, la composition du vaccin contient également un adjuvant.

Dans un autre aspect, la composition du vaccin contre la grippe contient un virus grippal atténué.

Selon une autre modalité, le vaccin contre la grippe comprend un vecteur comprenant un gène codant pour l'hémagglutinine d'un virus grippal.

De façon préférée, le vecteur est un poxvirus.

Dans un aspect particulier, le vecteur comprend également un gène codant pour la neuraminidase d'un virus grippal.

Selon un autre aspect, la composition du vaccin contient également un adjuvant.

Selon un mode de réalisation du procédé selon l'invention, l'hémagglutinine du virus de la grippe sous la forme de protéine et/ou de gène codant pour cette protéine contenue dans la composition du vaccin qui est administré aux poules et l'hémagglutinine du virus de la grippe qui est utilisée pour infecter la cavité allantoïque des oeufs embryonnés des poules vaccinées ont des sous types différents.

Selon un autre mode de réalisation, l'hémagglutinine du virus de la grippe sous la forme de protéine et/ou de gène codant pour cette protéine contenue dans la composition du vaccin qui est administré aux poules et l'hémagglutinine du virus de la grippe qui est utilisée pour infecter la cavité allantoïque des oeufs embryonnés des poules vaccinées ont le même sous type.

Selon encore un autre mode de réalisation, l'hémagglutinine du virus de la grippe sous la forme de protéine et/ou de gène codant pour cette protéine contenue dans la composition du vaccin qui est administré aux poules et l'hémagglutinine du virus de la grippe qui est utilisée pour infecter la cavité allantoïque des oeufs embryonnés des poules vaccinées sont identiques.

Selon encore un autre mode de réalisation, le virus de la grippe contenu dans la composition du vaccin qui est administré aux poules est identique au virus de la grippe qui est utilisé pour infecter la cavité allantoïque des oeufs embryonnés des poules vaccinées.

Dans un mode de réalisation particulièrement préféré, l'hémagglutinine du virus de la grippe sous la forme de protéine et/ou de gène codant pour cette protéine contenue dans la composition du vaccin qui est administré aux poules et l'hémagglutinine du virus de la grippe qui est utilisée pour infecter la cavité allantoïque des oeufs embryonnés des poules vaccinées ont le sous type H5, H6, H7 ou H9.

Dans un autre mode de réalisation particulièrement préféré, l'hémagglutinine du virus de la grippe sous la forme de protéine et/ou de gène codant pour cette protéine contenue dans la composition du vaccin qui est administré aux poules et l'hémagglutinine du virus de la grippe qui est utilisée pour infecter la cavité allantoïque des oeufs embryonnés des poules vaccinées ont le sous type H5 ou H7.

Dans un aspect particulier, le procédé selon l'invention comprend une étape supplémentaire de purification du virus.

Dans un autre aspect particulier, le procédé selon l'invention comprend une étape supplémentaire d'inactivation du virus.

Elle a également pour objet l'utilisation d'un procédé selon l'invention pour la fabrication d'un vaccin destiné à la prévention de la grippe.

Dans un aspect particulier, l'utilisation du procédé selon l'invention sert à la fabrication d'un vaccin destiné à la prévention de la grippe humaine pandémique.

Dans un autre aspect particulier, l'utilisation d'un procédé selon l'invention sert à la fabrication d'un vaccin destiné à la prévention de la grippe humaine épidémique.

Dans encore un autre aspect, l'utilisation d'un procédé selon l'invention sert à la fabrication d'un vaccin destiné à la prévention de la grippe chez les équidés, les suidés, les canidés, les félidés, les mustélidés et les espèces aviaires.

L'invention a aussi pour objet l'utilisation des oeufs de poules vaccinées contre la grippe pour la production d'un virus grippal.

Selon un aspect préféré l'utilisation des oeufs de poules vaccinées contre la grippe sert à la fabrication d'un vaccin contre la grippe.

Enfin selon un dernier aspect, l'utilisation des oeufs de poules vaccinées contre la grippe sert à la production d'un virus grippal ou à la fabrication d'un vaccin contre la grippe, selon laquelle les oeufs contiennent des anticorps dirigés contre l'hémagglutinine du virus de la grippe et de façon particulière les oeufs contiennent des anticorps dirigés contre l'hémagglutinine du virus de la grippe ayant le sous type H5, H6, H7 ou H9.

### Description détaillée de l'invention

Par « virus de la grippe ou virus grippal », on désigne aussi bien le virus grippal provenant d'une souche sauvage, que le virus grippal provenant d'une souche réassortante qui résulte du réassortiment des segments génomiques d'une ou plusieurs souches sauvages avec une souche « master » sélectionnée pour son fort potentiel de croissance dans l'oeuf. La souche réassortante acquiert des caractéristiques de la souche « master » mais conserve au moins les caractéristiques de l'HA et de la NA de la souche sauvage, ce qui signifie que l'identité entre la séquence protéique de l'HA et de la NA de la souche réassortante et la séquence protéique de l'HA et de la NA de la souche sauvage, déterminée au moyen d'un programme d'alignement global, est d'au moins 95 %, de façon préférée d'au moins 98 %, de façon plus préférée d'au moins 99 %, et de façon encore plus préférée de 100 %. La souche réassortante peut être obtenue par co-infection d'une cellule sensible avec la souche sauvage et la souche « master » suivie des moyens appropriés pour sélectionner la souche réassortante désirée. Elle peut également être obtenue par génétique inverse à partir des acides nucléiques de la souche sauvage et de la souche « master » et expression dans les systèmes d'expression multi-plasmidiques comme décrits dans WO 01/83794, WO 03/091401 et dans Proc. Nat. Acad. Sci. USA 96 :9345-9350 (1999). Dans le cas des souches hautement pathogènes, comme les souches H5 et H7 une modification du site de clivage comprenant de multiples acides aminés basiques pourra être réalisée de manière à rendre la souche réassortante faiblement pathogène.

Par commodité de langage, on utilise indifféremment le terme de souche vaccinale ou de virus vaccinal pour désigner le virus de la grippe servant à la fabrication du vaccin contre la grippe; de la même façon on utilise le terme de souche infectante ou de virus infectant pour désigner le virus de la grippe servant à infecter du matériel biologique (oeufs, animaux).

Par ailleurs, on utilise le terme d'oeufs de poules vaccinées pour désigner des oeufs de poules fécondés provenant de poules qui ont été préalablement vaccinées et mises en contact avec des coqs.

D'une façon générale, le vaccin contre la grippe servant à la vaccination des poules peut être fabriqué à partir de n'importe quelle souche de virus de la grippe. La souche vaccinale peut être un virus de type A, mais aussi de type B ou C. Lorsqu'il s'agit d'une souche de virus de type A, le virus peut avoir n'importe quel sous type de HA et/ou n'importe quel sous type de NA. Il peut s'agir, par exemple, de souches virales ayant le sous type H1N1 ou H3N2 actuellement responsables de la grippe humaine de type A épidémique.

L'intérêt à vacciner des poules avec un virus grippal est important dès lors que la vaccination confère une protection contre la grippe aviaire.

La grippe aviaire peut passer inaperçue ou se caractériser par un ensemble de manifestations, souvent d'ordre respiratoire et/ou intestinal, d'intensité plus ou moins grande, altérant plus ou moins l'état général des poules, pouvant aboutir à la mort de l'animal lorsque la souche de virus est hautement pathogène. La grippe aviaire entraîne souvent une diminution, voire une disparition de l'activité de ponte des oeufs. Les souches de virus faiblement pathogènes appartenant aux sous type H6N2 ou H9N2 ou même H5 ou H7 sont souvent responsables des formes légères de grippe aviaire, entrainant généralement une baisse ou une disparation de la production des oeufs mais pas de mortalité importante. Par contre, les souches de virus hautement pathogènes appartenant aux sous types H5 et H7 (notamment, H5N1, H5N2, H5N9, H7N1, H7N4 ou H7N7) sont très virulentes et causent une mortalité très importante dans les élevages de poules.

L'HA est un antigène essentiel dans le développement d'une immunité protectrice contre la grippe. Le vaccin utilisé dans le procédé selon l'invention comprend dans sa composition au moins l'HA d'un des virus de la grippe sous la forme de protéine et/ou de gène codant pour cette protéine.

Par « gène » on entend une séquence nucléotidique correspondant à une phase ouverte de lecture et codant pour une protéine. Le gène sous la dépendance de séquences régulatrices de l'expression (promoteur, enhancer, signal de polyadenylation, arrêt de transcription...) est inséré dans l'acide nucléique d'un vecteur, notamment un plasmide ou un virus, ces séquences régulatrices pouvant être celles qui sont habituellement associées à la phase ouverte de lecture (séquences endogènes) ou provenir d'une origine différente (séquences exogènes).

Il peut s'agir de l'HA d'une souche de virus de la grippe humaine mais qui n'est pas pathogène pour les poules. De façon préférée, il contient une HA d'intérêt, c'est-à-dire une HA qui a le même sous type que l' HA d'une souche virale qui est responsable d'une grippe aviaire et contre laquelle on cherche à immuniser et à protéger les poules. De façon préférée, le degré d'identité entre la séquence protéique de l'HA présente dans le vaccin et celle de la souche contre laquelle on veut protéger les poules est d'au moins 80 %, de façon préférée d'au moins 90 % et de façon encore plus préférée d'au moins 95 %, déterminée au moyen d'un programme d'alignement global (tel que le programme Blast).

Classiquement le vaccin comprenant l'HA est sous la forme d'une composition contenant du virus grippal entier inactivé, ou un produit dérivé du virus grippal entier inactivé.

Par «produit dérivé du virus entier inactivé » on entend une composition vaccinale inactivée (c'est-à-dire non infectieuse) qui est préparée à partir d'une souche de virus et qui comprend au moins l'HA de la dite souche de virus. Le produit dérivé d'une souche de virus entier peut être du virus fragmenté (ou splitté) et dans ce cas on parle de vaccin « splitté ». Un autre produit dérivé d'une souche de virus entier est l'HA de cette souche en tant que telle ou associée à la NA qui a été obtenue en utilisant des procédés d'extraction et de purification et dans ce cas on parle de vaccin « sous unitaire ». L'HA peut aussi être intégrée secondairement dans un virosome. La composition vaccinale contenant du virus grippal entier inactivé, ou un produit dérivé du virus entier inactivé peut contenir également un ou plusieurs adjuvants ou formulations adjuvantes. Comme exemple de formulations adjuvantes non limitatives on cite les émulsions eau-dans-huile ou huile-dans-eau, comme l'émulsion MF59^{®} (Vaccine Design - The Subunit and Adjuvant Approach Edited by M. Powell and M. Newman, Plenum Press, 1995 page 183), les formulations à base de liposomes, des formulations à base de MPL (Vaccine Design - The Subunit and Adjuvant Approach Edited by M. Powell and M. Newman, Plenum Press, 1995 pages 1186-187), d' Avridine (Vaccine Design - The Subunit and Adjuvant Approach Edited by M. Powell and M. Newman, Plenum Press, 1995 page 148), de Dimethyldioctadecylammonium bromide (Vaccine Design - The Subunit and Adjuvant Approach Edited by M. Powell and M. Newman, Plenum Press, 1995 page 157), de *Corynebacterium parvum*, de saponine, de lysolecithin, de dérivés pluroniques (Hunter H. et coll. 1991, vaccine, 9: 250-256) (Vaccine Design - The Subunit and Adjuvant Approach Edited by M. Powell and M. Newman, Plenum Press, 1995 page 200 et pages 297-311)), de sels d'aluminium ou de combinaisons de celles-ci (Vaccine Design - The Subunit and Adjuvant Approach Edited by M. Powell and M. Newman, Plénum Press, 1995 pages 249-276). De manière préférée les emulsions eau-dans-huile sont composées d'huile de paraffine, d'un tensio-actif hydrophile tel que le polysorbate 80, polysorbate 85 et d'un tensio actif lipophile tel que l'oleate de sorbitan, le trioleate de sorbitan. Des exemples d'émulsions utilisées chez la poule sont décrits dans Stone et coll. (1983, Avian Dis., 27 : 688-697 ; 1993, Avian Dis., 37: 399-405 ; 1991, Avian Dis., 35: 8-16) ; dans M. Brugh et coll. (1983, Am. J ; Vet. Res., 44: 72-75) ; dans Woodward L. et coll. (1985, Vaccine, 3: 137-144) ; dans (Vaccine Design - The Subunit and Adjuvant Approach Edited by M. Powell and M. Newman, Plenum Press, 1995 page 219). La souche vaccinale provient généralement d'une souche sauvage qui a été isolée chez la poule, la dinde, le canard, l'oie, ou autre espèce aviaire, cette souche étant le plus souvent faiblement pathogène pour la poule. Comme exemple d'isolats (souches sauvages) servant à la préparation de vaccins pour protéger les poules contre la grippe aviaire de sous-type H5 ou H7, on cite les isolats A/turkey/Wisconsin/68 ou A/chicken/Italy/22A/98 qui sont des souches virales ayant le sous type H5N9, les isolats A/turkey/England/N-28/73, A/chicken/Mexico/238/94/CPA, A/chicken/Mexico/232/94/CPA ou A/duck/Potsdam/1402/86 qui sont des souches virales ayant le sous type H5N2, l'isolat A/goose/Guandong/1/1996 qui est une souche virale HP ayant le sous type H5N1, les isolats A/chicken/Italy/AG-473/1999, ou A/chicken/Italy/1067/1999 qui sont des souches virales ayant le sous type H7N1, l'isolat A/chicken/Pakistan/95 qui est une souche HP ayant le sous-type H7N3, l'isolat A/duck/Postdam/15/80 qui est une souche virale ayant le sous type H7N7. Comme exemple de souches virales H9N2 utilisées pour protéger les oiseaux domestiques contre la grippe aviaire de sous type H9 on cite les isolats A/chicken/Iran/AV12221/98 et A/chicken/UAE/415/99. Comme exemple de souche virale H6N2 utilisée pour protéger les poules contre la grippe aviaire de sous type H6 on cite l'isolat A/turkey/Italy/90). Les souches vaccinales servant à la fabrication de vaccins peuvent également être des réassortants de souches sauvages obtenus notamment par génétique inverse. On cite notamment la souche vaccinale Re-1 qui est une souche réassortante obtenue par recombinaison inverse de la souche sauvage H5N1 A/goose/Guandong/1/96 avec la souche « master » A/PR/8/34 qui se reproduit très bien dans les oeufs. (Tian et coll., 2005, Virology, 341: 153-162). Un autre exemple de souche réassortante obtenue par génétique inverse est la souche H5N3 obtenu par réassortiment génétique et contenant l'hémagglutinine H5 de la souche H5N1 A/chicken/Vietnam/C58/04, la neuraminidase de la souche H2N3 A/duck/Germany/1215/73, et les gène internes de la souche « master » A/PR/8/34 (Webster et coll., 2006, Virology , 351: 301-311).

Les vaccins contre la grippe aviaire contiennent habituellement le virus d'une seule souche de virus grippal inactivée (vaccins monovalents) mais dans certains cas il peut être avantageux d'utiliser des vaccins multivalents contenant plusieurs souches de virus grippal inactivées. Il s'agit notamment d'un vaccin à base de souches H7N1 et H5N9 qui est une émulsion eau-dans-huile contenant les souches vaccinales inactivées A/chicken/Italy/22A/98 (H5N9) et A/chicken/Italy/1067/1999 (H7N1). Les vaccins inactivés peuvent également contenir des valences différentes par exemple, un vaccin bivalent contre la grippe aviaire H9N2 et la maladie de Newcastle. Les vaccins inactivés sont généralement administrés par voie parentérale (intramusculaire ou sous-cutanée). On peut aussi les administrer sous forme de spray comme dans le cas du vaccin Aerovac AI commercialisé par Investigacion Aplicada qui contient la souche vaccinale inactivée A/chicken/Mexico/232/94/CPA (H5N2). Le schéma d'immunisation comprend habituellement une ou deux administrations à 2 à 4 semaines d'intervalle. La dose vaccinale administrée varie en fonction de l'âge des animaux mais contient habituellement l'équivalent de 10 à 200 µl de liquide allantoidien titrant 10⁸ à 10¹⁰ EID₅₀/ml avant inactivation. La dose vaccinale est habituellement administrée sous un volume variant entre 0,05 et 1 ml. La préparation de vaccins inactivés contre la grippe aviaire est décrite par Stone H. (1987, Avian Dis. 31: 483-490). Dans la mesure où le sous type de l' HA de la souche vaccinale est le même que celui de l'HA de la souche responsable de la grippe aviaire pathogène, et sur la base d'un degré d'identité entre les séquences protéiques des deux HA de l'ordre de 80 à 90 % déterminé au moyen d'un programme d'alignement global, le taux de protection obtenu contre les symptômes cliniques de la grippe aviaire (morbidité et mortalité) est généralement de plus 80 % et de façon préférée de plus de 90%. Ceci est confirmé par les travaux de Bublot M. et coll., 2007, Avian Dis. 51: 332-337, qui montrent qu'un degré d'identité de l'ordre de 80 à 90 % entre la séquence protéique de l' HA de la souche vaccinale et la séquence protéique de l' HA de la souche infectante pathogène suffit pour obtenir ce taux de protection. De plus, il n'est pas nécessaire que le sous type de la NA de la souche vaccinale soit le même que celui de la NA de la souche infectante pathogène pour obtenir ce taux de protection. Par ailleurs les vaccins contre la grippe aviaire diminuent fortement l'excrétion du virus chez les animaux vaccinés et éprouvés avec du virus infectieux mis en évidence par une diminution très nette de la charge virale observée dans des prélèvements oraux et cloacaux (Bublot M. et coll., 2007, Avian Dis. 51: 332-337). Un autre effet bénéfique des vaccins contre la grippe aviaire est de diminuer la diffusion du virus dans les élevages de poules.

Selon un autre mode de réalisation du procédé selon l'invention, le vaccin servant à l'immunisation des poules est sous la forme d'une composition contenant une souche de virus grippal atténuée. La souche vaccinale est généralement sous la forme d'un réassortant qui a été sélectionné à la suite d'un réassortiment génétique entre une souche sauvage exprimant l'HA d'intérêt et accessoirement la NA d'intérêt et une souche « master » qui a été adaptée au froid et/ou qui est sensible à la température. Le réassortant est une souche virale qui exprime à sa surface l'HA et accessoirement la NA d'intérêt tout en conservant les caractéristiques phénotypiques de la souche « master » qui concernent sa capacité à se répliquer uniquement dans des limites étroites de température, inférieures à celle de la température interne des oiseaux. De ce fait, la souche vaccinale après avoir été administrée aux poules, se réplique de façon limitée et localement. Les procédés d'obtention de ces souches réassortantes sont bien connues de l'homme du métier et sont décrits notamment dans WO 03/091401, WO 2006/063053 et par Wareing M.D. et coll., 2002, Vaccine 20: 2082-2090. L'administration du vaccin se fait généralement par nébulisation. Un autre moyen de produire une souche atténuée est de tronquer le gène codant pour la protéine NS1 (Richt J.A. et coll., Vaccination of pigs against swine influenza viruses by using an NS1-truncated modified live-virus vaccine, 2006, J. Virol., 80: 11009-18 ; Quinlivan M. et coll., Attenuation of equine influenza viruses through truncations of the NS1 protein, 2005, J Virol., 79: 8431-9).

Les souches vaccinales peuvent être produites par tous moyens, en mettant en oeuvre des techniques de culture sur cellules telles que les cellules Vero, les cellules MDCK, les cellules PER.C6, ou les cellules d'embryons de poulets (CEK, PCJ)) et/ou en utilisant les procédés classiques de production sur oeufs embryonnés. Les procédés de récolte, de purification et selon les cas d'inactivation du virus sont également bien connus de l'homme du métier.

Selon un autre mode de réalisation du procédé selon l'invention, le vaccin est sous la forme de protéines produites dans un système d'expression in vitro. Par exemple, l'HA peut être produite en système d'expression utilisant un baculovirus recombiné en cellules d'insecte. (Crawford J. et coll., Baculovirus-derived hemagglutinin vaccines protect against lethal influenza infections by avian H5 and H7 subtypes, 1999, Vaccine, 17: 2265-74). L'hémagglutinine peut être également exprimée in vitro sous forme de "virus-like particles" (VLPs) (Prel A. et coll., Assessment of the protection afforded by triple baculovirus recombinant coexpressing H5, N3, M1 proteins against a homologous H5N3 low-pathogenicity avian influenza virus challenge in Muscovy ducks, 2007, Avian Dis., 51: 484-9 ; Pushko P. et coll., Influenza virus-like particles comprised of the HA, NA, and M1 proteins of H9N2 influenza virus induce protective immune responses in BALB/c mice, 2005, Vaccine, 23: 5751-9) ou de pseudotype à base de rétrovirus (Szecsi J. et coll., 2006, Virol. J., 3 : 70). Les protéines produites *in vitro* ou les particules virales produites sont plus ou moins purifiées et ensuite adjuvées avec différents adjuvants, tels ceux utilisés avec les vaccins inactivés.

Selon un autre mode de réalisation du procédé selon l'invention, le vaccin contre la grippe comprend un vecteur comprenant un fragment d'acide nucléique codant pour l'HA du virus de la grippe.

Le terme de vecteur fait référence à des structures d'acides nucléiques qui peuvent être propagées et/ou transférées à des organismes, des cellules ou des composants cellulaires. Cela inclut notamment les plasmides, les virus, les bactériophages, les pro-virus, les phagemides, les chromosomes artificiels qui sont capables de se répliquer de façon autonome ou qui peuvent s'intégrer dans le chromosome d'une cellule hôte.

Par « vecteur comprenant le gène codant pour l'HA et/ou la NA du virus de la grippe », on entend un vecteur comprenant l'acide nucléique codant pour l'HA et/ou la NA d'intérêt et qui après introduction dans une cellule aviaire exprime l'HA et/ou la NA dans cette cellule. Il peut s'agir d'un plasmide exprimant l'HA d'intérêt, mais généralement il s'agit d'un vecteur viral contenant dans son génome l'acide nucléique codant pour l'HA d'intérêt et exprimant l'HA d'intérêt dans les cellules infectées. L'intégration dans le génome du vecteur viral de l'acide nucléique codant pour l'HA se fait généralement par des techniques de biologie moléculaire notamment la recombinaison génétique, le clonage, la génétique inverse. L'HA peut ou non être exprimée à la surface du vecteur viral. De préférence, le vecteur viral a été atténué de manière classique par de multiples passages in vitro ou par délétion de certains gènes pour que la réplication du virus vecteur dans les cellules aviaires soit suffisamment limitée et sans conséquence sur l'état général des poules et considéré ainsi comme étant non pathogène. Comme exemple de vecteurs viraux, on cite les paramyxovirus aviaires (Ge J., et coll., Newcastle disease virus-based live attenuated vaccine completely protects chickens and mice from lethal challenge of homologous and heterologous H5N1 avian influenza viruses, 2007, J Virol., 81: 150-8) ; l'herpesvirus de la dinde (HVT) ou de la maladie de Marek (Sondermeijer et coll. 1993, Vaccine, 11, 349-358); le virus de la laryngotrachéite infectieuse (ILTV) (Veits J., et coll., Deletion of the non-essential UL0 gene of infectious laryngotracheitis (ILT) virus leads to atténuation in chickens, and UL0 mutants expressing influenza virus haemagglutinin (H7) protect against ILT and fowl plague, 2003, J Gen. Virol., 84: 3343-52; Luschow D., et coll., Protection of chickens from lethal avian influenza A virus infection by live-virus vaccination with infectious laryngotracheitis virus recombinants expressing the hemagglutinin (H5) gene, 2001, Vaccine, 19: 4249-59); les adénovirus (Francois A., et coll., Avian adenovirus CELO recombinants expressing VP2 of infectious bursal disease virus induce protection against bursal disease in chickens, 2004, Vaccine, 22: 2351-60; Gao W., et al., Protection of mice and poultry from lethal H5N1 avian influenza virus through adenovirus-based immunization, 2006, J Virol., 80: 1959-64; Toro H., et coll., Protective avian influenza in ovo vaccination with non-replicating human adenovirus vector, 2007, Vaccine, 25: 2886-91); les coronavirus (Cavanagh, 2007, Vet Res. 38: 281-97; Eriksson, 2006, Clin. Dev. Immunol. 13: 353-60) mais de préférence on utilise pour vacciner les poules, les poxvirus, notamment le virus de la vaccine, NYVAC (virus de la vaccine délété), le virus de la vaccine souche MVA, particulièrement les avipox notament, le canary pox, ALVAC (canary pox atténué), le pigeon pox, le quail pox, le turkey pox, le sparrow pox et tout particulièrement le fowl pox, TROVAC (fowlpox atténué) qui sont décrits notamment dans AU 701599B, AU 701781B et dans US 5,756,103. Selon les cas, les vecteurs viraux atténués expriment seulement la HA d'intérêt : il s'agit notamment du vaccin contenant un vecteur fowlpox TROVAC exprimant l'HA de la souche de virus grippal H5N8 (A/turkey/Ireland/1378/83). Dans d'autres cas, le vecteur viral atténué exprime à la fois la HA d'intérêt en association avec une NA d'intérêt comme le poxvirus recombinant décrit dans 2003, Avian Pathology, 32: 25-32 qui exprime à la fois la HA et la NA provenant d'une souche de virus grippal H5N1. Une NA d'intérêt est une NA qui a habituellement le même sous type que la NA de la souche virale contre laquelle on cherche à immuniser et protéger les poules Dans d'autres cas encore, le vecteur atténué exprime plusieurs HA d'intérêt appartenant à des sous types différents comme dans le cas du poxvirus recombinant décrit par MingXiao M. et coll, 2006, Vaccine, 24: 4304-4311 qui exprime à la fois les sous types H5 et H7. Le pouvoir immunogène de ces vecteurs peut être encore renforcé en y introduisant les gènes codant pour des cytokines, chemokines, exerçant un pouvoir immunostimulant comme l'IL-1, IFN, CSF, GM-CSF, IL-2, IL-12, IL-18, TNF 5 (Vaccine, (2006), 24: 4304-4311). Les vaccins à base de vecteurs codant pour l'HA du virus de la grippe peuvent également être adjuvés pour augmenter leur immunogénicité.

Les compositions vaccinales contenant des vecteurs viraux peuvent être administrées par différentes voies qui dépendent notamment du vecteur : par exemple, transfixion de la membrane alaire (vecteur poxvirus), par voie intramusculaire, sous cutanée ou transdermale avec ou sans aiguille (tout vecteur), par voie *in ovo* (dans l'oeuf embryonné de 17 à 19 jours ; par exemple, vecteur HVT/Marek et adenovirus), par voie oculaire, oro-nasale, par spray ou dans l'eau de boisson (vecteur paramyxovirus, coronavirus, adénovirus), en une ou deux injections séparées par un intervalle d'au moins 15 jours. La (les) doses vaccinale(s) administrée(s) sont de l'ordre de 1 à 7 log₁₀ unité infectieuse 50 % avec une préférence pour une dose de 2 à 4 log₁₀ pour les vecteurs fowlpox. L'intérêt d'une vaccination à base d'un vecteur viral par rapport à une vaccination classique utilisant du virus grippal entier inactivé ou atténué réside en ce que l'on peut distinguer les animaux vaccinés des animaux infectés. De plus, la vaccination avec un vecteur viral favorise le développement d'une immunité cellulaire pouvant renforcer la protection des animaux. Comme cela est illustré dans Avian Dis., (2007), 51: 325-331 et Avian Dis., (2007), 51: 498-500, le degré de protection obtenu chez les poules et la diminution de la diffusion du virus dans les élevages de volailles sont du même ordre que ce que qu'on observe avec un vaccin classique renfermant du virus grippal inactivé. Dans les cas où la vaccination des poules comporte plusieurs injections, le vaccin utilisé lors de la première administration peut être différent de celui utilisé lors de la deuxième injection ou les suivantes. On peut utiliser deux vecteurs viraux atténués différents comme par exemple utiliser un vecteur ALVAC recombinant exprimant l'HA d'intérêt lors de la première immunisation et un vecteur TROVAC ou NYVAC recombinant exprimant la même HA d'intérêt lors des immunisations suivantes de façon à ce que la réponse anticorps dirigée contre le vecteur ALVAC n'empêche pas l'infection des cellules de poules par le vecteur recombinant TROVAC ou NYVAC recombinant et par conséquent l'expression de l'HA dans les cellules infectées. On peut utiliser aussi la méthode dite du « prime boost » qui consiste à utiliser lors de la première injection un vecteur viral atténué exprimant une HA et à utiliser lors de la (les) injection(s) de rappel, un vaccin contenant par exemple, une ou plusieurs souches vaccinales inactivées appartenant au même sous type que l'HA utilisée lors de la première vaccination, ou bien procéder selon un ordre inverse. On peut enfin procéder à une vaccination ADN lors de la première injection en utilisant un plasmide exprimant l'HA d'intérêt, suivi d'injections de rappel en utilisant un vaccin contenant une souche vaccinale inactivée et/ou un vecteur viral atténué qui expriment une HA appartenant au même sous type que l'HA utilisée lors de la première vaccination ou qui est identique à l'HA utilisée lors de la première vaccination.

Quelque soit le type de vaccin administré ou le schéma de vaccination adopté, la protection des poules contre la grippe aviaire est assurée assez rapidement, généralement dans un délai de 7 à 18 jours après l'administration d'une dose vaccinale. Néanmoins pour assurer une protection des poules contre la grippe aviaire pendant toute la durée de leur activité de ponte qui dure environ un an, on recommande une à deux vaccinations de rappel qui se fait dans un délai de 3 à 16 semaines après la première administration vaccinale. Plusieurs schémas de vaccination avec des vaccins inactivés peuvent être utilisés chez les poules pondeuses : par exemple, 2 injections, la première à l'âge de 3 à 6 semaines et la seconde à l'âge de 16 à 19 semaines juste avant l'entrée en ponte, ou 3 injections, la première vers 2 à 4 semaines, la deuxième 3 à 4 semaines plus tard et la troisième à l'âge de 16 à 19 semaines juste avant l'entrée en ponte. Un rappel peut également être administré en cours de ponte. Dans le schéma « prime-boost » utilisant 2 vaccins différents, les poussins peuvent être vaccinés à 1 jour d'âge avec un vaccin à base de vecteur fowlpox ; ils reçoivent ensuite une (à l'âge de 16 à 19 semaines juste avant l'entrée en ponte) ou deux (à l'âge de 3 à 6 semaines et à l'âge de 16 à 19 semaines juste avant l'entrée en ponte) vaccinations avec un vaccin contenant du virus grippal inactivé.

Dans la mise en oeuvre du procédé selon l'invention, on prélève de préférence les oeufs des poules une fois que la protection des poules contre la grippe aviaire est assurée, ce qui se produit habituellement dans un délai de 7 à 18 jours après l'administration du vaccin (Bublot M., et coll. (2006, Annals of the New York Academy of Sciences 1081: 193-201); Van der Goot et coll. (2005, Proc. Natl. Acad. Sc., 102 : 18141-6) ; Ellis et coll. (Avian Pathol. 2004, 33, 405-412).

Malgré la présence d'anticorps antigrippaux dans les oeufs des poules vaccinées, notamment d'anticorps dirigés contre l'HA, et en particulier d'anticorps inhibant l'hémagglutination (IHA) qui bloquent la pénétration du virus grippal dans les cellules sensibles, le procédé utilisé pour produire du virus grippal à partir d'oeufs embryonnés provenant de poules vaccinées contre la grippe est classique. On utilise des oeufs embryonnés de 9 à 14 jours, provenant de poules vaccinées qui ont été élevées, de préférence, dans un environnement contrôlé. Le processus d'embryogénèse est contrôlé de la manière suivante : on utilise des oeufs qui ont été conservés à une température comprise entre 10 et 20°C, de préférence entre 16 et 18°C, pendant une durée qui n'excède pas en général une semaine après la ponte. On déclenche le processus d'embryogénèse en incubant les oeufs à une température de 37,5°C ± 1°C dans une enceinte humide ayant une humidité relative de 70 ± 10 % pendant une durée comprise entre 9 et 14 jours. Les oeufs embryonnés sont sélectionnés par mirage, et leurs cavités allantoïques sont infectées avec une dose de virus généralement comprise entre 2 et 7 log₁₀ TCID₅₀ sous un faible volume (environ 0.1 à 0.2 ml). On laisse le virus se multiplier pendant une durée allant généralement de 1 à 4 jours en fonction de la virulence de la souche de virus et à une température qui peut varier également en fonction du phénotype de la souche virale et de son degré d'adaptation au froid ou au chaud. La température de multiplication du virus grippal se situe généralement dans un intervalle allant de 28 à 39°C et de façon habituelle dans un intervalle de température allant de 33 à 39°C. Les liquides allantoïques infectieux sont alors récoltés et traités en fonction des utilisations que l'on souhaite en faire.

Ce procédé peut servir à produire un virus grippal dont le sous type de l'HA est différent du sous type de l'HA contenue dans le vaccin qui a servi à vacciner les poules. Il s'agit, par exemple, du cas, où les poules sont vaccinées avec un virus inactivé H5N9 pour protéger les poules contre la grippe aviaire H5 tandis que les oeufs de ces poules sont infectés avec un virus grippal H1N1 ou H3N2, ou même un virus grippal de type B pour préparer un vaccin contre les formes épidémiques de la grippe humaine actuelle. Selon une variante du procédé selon l'invention, le sous type de la HA du virus grippal qui est produit en utilisant les oeufs embryonnés de poules vaccinées a un sous type différent de l'HA qui a servi à vacciner les poules mais par contre la NA du virus grippal produit a le même sous type que la NA du virus utilisé dans le vaccin. Il s'agit par exemple du cas où les poules sont vaccinées avec une souche de virus inactivée H5N1 ou un poxvirus recombinant exprimant H5 et N1 pour protéger les poules contre la grippe aviaire tandis que les oeufs embryonnés des poules vaccinées sont infectés avec une souche de virus H1N1.

Selon une autre modalité du procédé selon l'invention, l'HA du virus grippal produit sur oeufs embryonnés de poules vaccinées a le même sous type que l'HA contenue dans le vaccin qui a servi à vacciner les poules. Il s'agit par exemple du cas où les poules sont vaccinées avec du virus inactivé H5N1 ou un poxvirus recombinant exprimant H5 tandis que les oeufs des poules vaccinées sont infectés avec un virus grippal H5N1 ou H5N9. Malgré la présence d'anticorps anti HA cross réactifs spécifiques « de sous type » dans l'oeuf (c'est le cas lorsque l'HA contenue dans la composition vaccinale a le même sous type que l'HA de la souche de virus grippal infectant les oeufs embryonnés de poules vaccinées), cela n'a pas d'effet négatif sur la réplication du virus.

Et de façon encore plus surprenante, cette réplication n'est pas non plus affectée dans le cas où il y a une identité parfaite entre le virus grippal qui est utilisé pour infecter les oeufs embryonnés de poules vaccinées et le virus grippal qui a été utilisé pour vacciner ces poules. Cela se traduit dans ce cas par la présence, dans les oeufs, d'un panel d'anticorps anti HA encore plus large puisque l'on retrouve à la fois des anticorps anti HA cross réactifs spécifiques de sous type et des anticorps anti HA très spécifiques de la souche (encore appelés anticorps spécifiques de souches (Voir exemple 3). Contrairement à l'opinion largement établie, la présence d'anticorps anti grippe dans l'oeuf et en particulier la présence d'anticorps anti HA n'affecte donc pas la réplication du virus grippal. Les quantités de virus et/ou d'antigène hémagglutinant récoltées dans les liquides allantoïques infectés provenant d'oeufs de poules vaccinées contre la grippe sont du même ordre que celles récoltées dans les liquides allantoïques infectés provenant d'oeufs de poules non vaccinées (voir exemples 1 et 2).

On peut mettre aussi à profit le procédé selon l'invention pour fabriquer une souche de virus réassortant. Dans ce cas, on co-infecte dans un premier temps des oeufs embryonnés de poules vaccinées avec une souche de virus sauvage et une souche de virus master qui se réplique bien dans les oeufs embryonnés comme par exemple la souche A/PR/8/34. Dans un deuxième temps on recueille les liquides allantoïques infectés contenant essentiellement un mélange de réassortants et la souche master tandis que la souche sauvage qui a de moins bonnes capacités à se répliquer est généralement en quantité très faible. On procède alors à la sélection de la souche réassortante exprimant à la fois les caractéristiques phénotypiques de la souche A/PR/8/34 (c'est-à-dire sa bonne aptitude à se répliquer dans les oeufs embryonnés) et l'HA ainsi que la NA de la souche sauvage par des étapes de clonages successifs en mélangeant à chaque étape de clonage le liquide allantoïque infectieux récolté avec des anticorps anti HA et anti NA spécifiques de A/PR/8/34 selon des méthodes bien connues de l'homme de métier. On peut aussi fabriquer une souche réassortante adaptée au froid et sensible à la chaleur dans la perspective d'un vaccin à virus vivant atténué. Dans ce cas on co-infecte dans un premier temps des oeufs embryonnés de poules vaccinées avec une souche de virus sauvage et une souche master qui a comme caractéristique phénotypique d'être adaptée au froid et sensible à la chaleur. Dans ce cas la température d'incubation des oeufs infectés se fait à une température plus basse que la normale (la température est souvent inférieure à 35°C, voire inférieure à 30°C). Dans un deuxième temps on recueille les liquides allantoïques infectés contenant essentiellement un mélange de réassortants et la souche master car la souche sauvage sensible au froid ne s'est pas répliquée. On procède alors à la sélection du réassortant exprimant à la fois les caractéristiques phénotypiques de la souche master (notamment l'adaptation au froid et/ou sa thermosensibilité) et la HA ainsi que la NA de la souche sauvage par des étapes de clonages successifs en mélangeant à chaque étape de clonage le liquide allantoïque infectieux récolté avec des anticorps anti HA et anti NA spécifiques de la souche master selon des méthodes bien connues de l'homme de métier.

Le procédé selon l'invention est mis en oeuvre en première intention pour préparer des vaccins destinés à protéger les élevages de poules et plus généralement les élevages d'oiseaux domestiques (canards, dindes, oies,...) contre la grippe aviaire. Les souches virales impliquées dans les formes asymptomatiques ou légères de grippe aviaire peuvent être de n'importe quel sous-type, et en particulier les sous types H9N2, H6N2, H7N2, H7N3 ou H7N1. Elles n'entrainent pas de mortalité importante dans les élevages mais peuvent être la cause d'une baisse transitoire de la production d'oeufs. Les souches virales hautement pathogènes impliquées dans les formes sévères de grippe aviaire entrainant une mortalité importante dans les élevages appartiennent généralement aux sous types H5 et H7, et en particulier, H5N1, H5N2, H5N8, H5N9, H7N1, H7N3, H7N4 ou H7N7.

Généralement, l'hémagglutinine du virus grippal contenue dans la composition vaccinale qui sert à vacciner les poules contre la grippe aviaire dans l'étape a) du procédé selon l'invention et l'hémagglutinine du virus qui sert à l'infection des oeufs embryonnés dans l'étape c) du procédé ont le même sous type et sont sélectionnées notamment parmi les sous types H5, H6, H7 et H9 car ce sont ceux que l'on retrouve principalement dans les souches de virus responsables de la grippe aviaire.

Dans un aspect particulier, l'hémagglutinine du virus grippal contenue dans la composition vaccinale qui sert à vacciner les poules contre la grippe aviaire dans l'étape a) du procédé selon l'invention et l'hémagglutinine du virus qui sert à l'infection des oeufs embryonnés dans l'étape c) du procédé ont le même sous type et sont sélectionnées parmi les sous types H5 et H7 car ce sont ceux que l'on retrouve dans les souches de virus responsables des formes sévères de grippe aviaire et de grippe humaine. Les souches de virus responsables des formes sévères de grippe aviaire et/ou de grippe humaine qui ont été caractérisées appartiennent généralement aux sous types H5N1, H5N2, H7N1, H7N3 ou H7N7.

Ainsi de façon très particulière, l'invention a pour objet :
Un procédé de production du virus de la grippe selon lequel :
   a. on vaccine des poules avec un virus grippal entier inactivé, selon lequel l'hémagglutinine du virus a le sous type H5 ou H7,
   b. on prélève les oeufs des poules vaccinées,
   c. on déclenche le processus d'embryogénèse,
   d. on infecte les oeufs embryonnés en introduisant dans la cavité allantoïque des oeufs embryonnés un virus grippal identique à celui qui a servi à la vaccination,
   e. on incube les oeufs embryonnés infectés dans des conditions de température et d'humidité qui permettent la réplication du virus et,
   f. on récolte le liquide allantoïque contenant le virus.

Lorsque le schéma de vaccination des poules prévoit deux injections, la première injection peut se faire avec une composition vaccinale qui contient à la place du virus grippal entier inactivé, un vecteur, généralement un poxvirus, comprenant le gène qui code pour le sous type H5 ou H7 de l'hémagglutinine du virus grippal.

Lorsque les liquides allantoïques infectés sont destinés à la production d'un vaccin contre la grippe, le procédé selon l'invention comprend généralement une étape supplémentaire de purification de la souche de virus et est éventuellement suivie ou précédée d'une étape d'inactivation virale en mettant en oeuvre des méthodes bien connues de l'homme de métier comme celles décrites dans FR 2201079 ou dans FR 1538322.

La purification peut être sommaire et se limiter à une étape de concentration du virus par centrifugation après avoir généralement clarifié les liquides allantoïques infectés. La purification peut être complétée par une étape de centrifugation zonale réalisée par exemple au moyen de gradients de densité de saccharose (EP 0 7760362). On peut aussi mettre en oeuvre des procédés chromatographiques pour purifier le virus. On obtient ainsi une suspension de virus entiers purifiés qui rentrent dans la composition des vaccins entiers inactivés ou de vaccins atténués.

L'inactivation de la suspension virale se fait par des moyens classiques, en utilisant de la β propiolactone (E. Budowsky et coll. 1991, Vaccine, 9: 319-325 ; 1991, Vaccine, 9: 398-402 ; 1993, Vaccine, 11: 343-348), éthylene imine ou dérivés (D. King 1991, Avian Dis. 35: 505-514) ou du formol (EP 0 776 0362).

La composition vaccinale à base de virus entiers et inactivés peut être formulée avec un ou plusieurs adjuvants. Bien que classiquement ces vaccins puissent être formulés avec des sels d'Aluminium ou dans une émulsion eau-dans-huile ou huile-dans-eau (cas des vaccins contre la grippe aviaire), on utilise habituellement une émulsion eau-dans-huile composée d'huile de paraffine, d'un tensio-actif hydrophile tel que le polysorbate 80, polysorbate 83, polysorbate 85 et d'un tensio-actif lipophile tel que l'oleate de sorbitan, le sesquioleate de sorbitan, le trioleate de sorbitan. On peut utiliser tout adjuvant susceptible d'augmenter la réponse humorale et /ou cellulaire contre la grippe. Comme exemple de formulations adjuvantes non limitatives on cite l'émulsion MF59^{®}, les formulations à base de liposomes, des formulations à base de MPL, de *Corynebacterium parvum*, de saponine, de lysolecithin, de dérivés pluroniques, ou des combinaisons de celles-ci.

La suspension de virus purifiée peut subir également des traitements ultérieurs. On obtient ainsi des produits dérivés du virus de la grippe. Par exemple, on peut fragmenter la suspension virale à l'aide d'agents détergents ou de solvants des lipides selon des méthodes bien connues de l'homme du métier pour fabriquer par exemple des vaccins à base de virus fragmentés ou splittés, des virosomes, ou des vaccins sous unitaires renfermant l'hémagglutinine du virus grippal. Les virus fragmentés ou splittés, les virosomes contenant l'hémagglutinine du virus grippal, les vaccins sous unitaires contenant l'hémagglutinine du virus grippal qui sont obtenus à partir du virus purifié sont considérés comme des produits dérivés du virus de la grippe. Les vaccins contenant ces produits dérivés peuvent être formulés de la même façon avec un ou plusieurs adjuvants.

Les vaccins obtenus au moyen du procédé selon l'invention sont destinés à protéger l'homme et l'animal contre la grippe.

Dans le domaine vétérinaire, le vaccin est principalement utilisé dans le domaine de la prévention de la grippe aviaire mais il peut être utilisé aussi pour prévenir ou réduire les symptômes de grippe et/ou l'excrétion virale chez les équidés, notamment le cheval, les canidés, notamment le chien, les félidés, notamment le chat, les suidés, notamment le porc, les mustélidés, notamment le vison, le furet, les espèces aviaires, notamment la poule, le canard, la dinde, la caille, la pintade, l'oie, l'autruche. Lorsque la composition vaccinale renferme une souche de virus entier inactivé ou un produit dérivé elle est généralement administrée par voie sous-cutanée ou intramusculaire, ou éventuellement sous forme de nébulisât dans les élevages de volailles. Lorsque le vaccin est sous la forme d'un virus vivant atténué, il est généralement administré par voie oro-nasale, spray, eau de boisson ou goutte dans l'oeil. Le schéma de vaccination prévoit généralement une injection ou une injection suivie d'un rappel. La dose vaccinale administrée dépend de la taille et de l'âge de l'animal. Elle contient habituellement entre 20 à 200 µl de liquide allantoidien titrant 10⁸ à 10¹⁰ EID₅₀/ml avant inactivation, injectée sous un volume compris entre 0.05 et 1 ml.

Chez l'homme, le vaccin est utilisé dans le domaine de la prévention de la grippe épidémique et de la grippe pandémique. Alors que la grippe épidémique affecte une population humaine déjà sensibilisée par contact (par infection) ou par vaccination avec une (des) souche(s) de virus grippale(s) pour laquelle il existe une parenté antigénique avec l'HA de la souche de virus responsable de l'épidémie et dans laquelle il existe une certaine immunité, même si elle n'est que partiellement efficace, la grippe pandémique affecte une population humaine non sensibilisée à une nouvelle souche de virus parce que l'HA de cette nouvelle souche n'a pas ou trop peu de parenté antigénique avec les souches de virus antérieures circulantes.

Le vaccin contre la grippe épidémique est destiné à protéger la population humaine contre des formes de grippe saisonnières occasionnées par des souches de virus grippales circulantes saisonnières ayant une parenté antigénique avec des souches de virus antérieures ayant déjà circulées. Actuellement les souches de virus grippal responsables de la grippe épidémique qui sont des souches épidémiques de virus grippal sont de type A et appartiennent aux sous types H1N1 ou H3N2 ou sont de type B.

Le vaccin contre la grippe pandémique est destiné à protéger la population humaine contre l'infection par une souche pandémique de virus grippal qui est une nouvelle souche sans parenté antigénique au niveau de l'HA avec des souches de virus antérieures circulantes.

Le vaccin contre la grippe épidémique ou pandémique peut être sous la forme de vaccin vivant atténué ou de vaccin inactivé, bien que l'on préfère un vaccin inactivé pour la prévention de la grippe pandémique. Le vaccin peut être sous la forme de vaccin monovalent (vaccin préparé à partir d'une seule souche de virus grippal) ou de vaccin multivalent (vaccin préparé à partir de plusieurs souches de virus grippal). La composition du vaccin contre la grippe épidémique est actuellement sous la forme de vaccin trivalent préparé à partir des souches H3N2, H1N1 et d'une souche de virus de type B. Le vaccin inactivé est généralement sous la forme de virus entier, de virus fragmenté (virus splitté), de virosomes, ou sous une forme sous unitaire renfermant de l'HA et éventuellement renferme un ou plusieurs adjuvants comme ceux précédemment cités. Tandis que le vaccin vivant atténué est généralement administré par voie orale ou nasale pour favoriser le développement d'une immunité mucosale, le vaccin inactivé peut être administré par voie parentérale (voie intramuculaire ou sous cutanée), par voie intradermique ou même par voie mucosale (voie intranasale) ou même en combinant deux voies d'administration différentes comme décrit dans WO 01/22992. Le schéma de vaccination prévoit généralement une injection ou une injection suivi d'un rappel. La dose vaccinale administrée dépend de l'âge de la personne et de la présence ou non d'un adjuvant. Classiquement la dose vaccinale contient l'équivalent de 15 µg d'HA de chaque souche vaccinale contenue dans le vaccin. Cette dose peut être abaissée jusqu'à environ 1 à 2 µg d'HA lorsque le vaccin est adjuvé, ou augmentée à 30 µg d'HA voire plus chez la personne âgée ou atteinte d'un déficit immunitaire.

L'invention a enfin pour objet :
L'utilisation des oeufs de poules vaccinées contre la grippe pour la production de virus grippaux ou pour la fabrication d'un vaccin contre la grippe. Contrairement à l'opinion largement établie, la présence d'anticorps dirigés contre l'hémagglutinine du virus grippal dans les oeufs des poules vaccinées n'affecte pas la quantité de virus grippal qui est produit après avoir infecté leurs cavités allantoïques.

Les exemples qui suivent illustrent de façon non limitative différents modes de réalisation de l'invention.
La figure 1 représente la séquence nucléotidique complète du plasmide donneur pJY 1394.1
La figure 2 représente la séquence nucléotidique de l'insert dans le plasmide donneur pJY1394.1 comportant les bras flanquants le locus d'insertion F8, ainsi que le promoteur vaccine H6 suivi du gène synthétique modifié au niveau du site de clivage codant pour l'HA de la souche A/chicken/Indonesia/7/03.

Les différentes origines de ces séquences sont les suivantes :
- De 1 à 53 : séquence partielle du plasmide de clonage comprenant la séquence du primer M13F (soulignée)
- De 54 à 1483 : séquence du « bras gauche » flanquant le locus d'insertion F8 dans le génome du vecteur TROVAC fowlpox.
- De 1484 à 1568 : séquence de liaison entre le bras gauche et le promoteur H6
- De 1569 à 1692 : séquence du promoteur H6 du virus de la vaccine
- De 1693 à 3387 : séquence du gène synthétique modifié HA de la souche A/chicken/Indonesia/7/03 ; la séquence en acide aminés est indiquée au dessus de la séquence nucléotidique en utilisant le code 1 lettre par acide aminé
- De 3388 à 3414 : séquence de liaison entre le gène HA et le bras droit comprenant une séquence TTTTTAT d'arrêt de transcription des gènes précoces du fowlpox
- De 3415 à 4790 : séquence du « bras droit » flanquant le locus d'insertion F8 dans le génome du vecteur TROVAC fowlpox.
- De 4791 à 4885 : séquence partielle du plasmide de clonage comprenant la séquence du primer M13R (soulignée)

### Exemple 1 : Procédé de production de deux souches vaccinales A/New Caledonia/20/99 (H1N1) et A/New York/55/04 (H3N2) servant à la préparation de vaccins contre la grippe épidémique humaine à partir d'oeufs embryonnés de poules qui ont été vaccinées, soit par deux injections d'un vaccin inactivé contenant une souche de virus grippal aviaire A/chicken/Italy/22A/98 (H5N9), soit par une injection d'un avipoxvirus recombinant codant pour l'HA d'une souche H5N1 suivi d'une deuxième injection d'un vaccin inactivé contenant une souche de virus grippal aviaire A/chicken/Italy/22A/98 (H5N9).

### 1.1) Protocole opératoire

### 1.1.1) Construction du vecteur recombinant vFP2211

Le vFP2211, est un virus fowlpox recombinant, dans le génome duquel a été inséré un gène synthétique codant pour l'hémagglutinine (HA) de la souche H5N1 A/chicken/Indonesia/7/03. Le gène HA a été synthétisé de manière à obtenir un cadre ouvert de lecture qui code pour une séquence en acide aminé identique à la séquence native de la souche A/chicken/Indonesia/7/03 décrite dans la base de données de séquences nucléotidique GenBank sous la référence EF473080 (ou de séquences protéiques GenPept sous la référence ABO30346) à l'exception du site de clivage. La séquence d'acides aminés RERRRKKRG situés entre les positions 339 et 347 (correspondant au site de clivage d'une souche hautement pathogène) a été remplacée par la séquence RE----TRG. Ce site de clivage ainsi modifié correspond au site de clivage des souches faiblement pathogènes de sous-type H5.

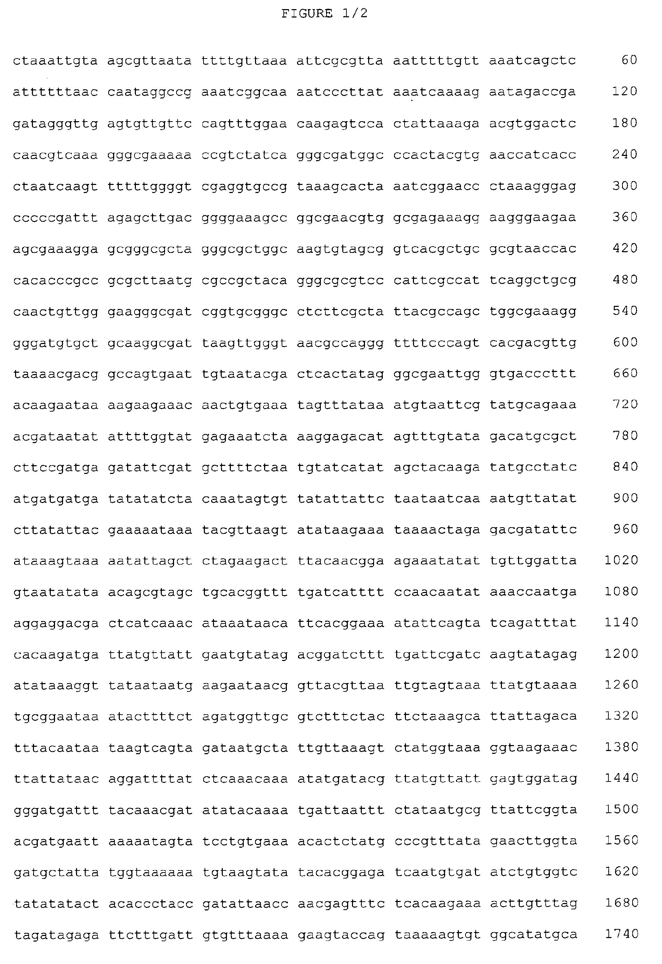

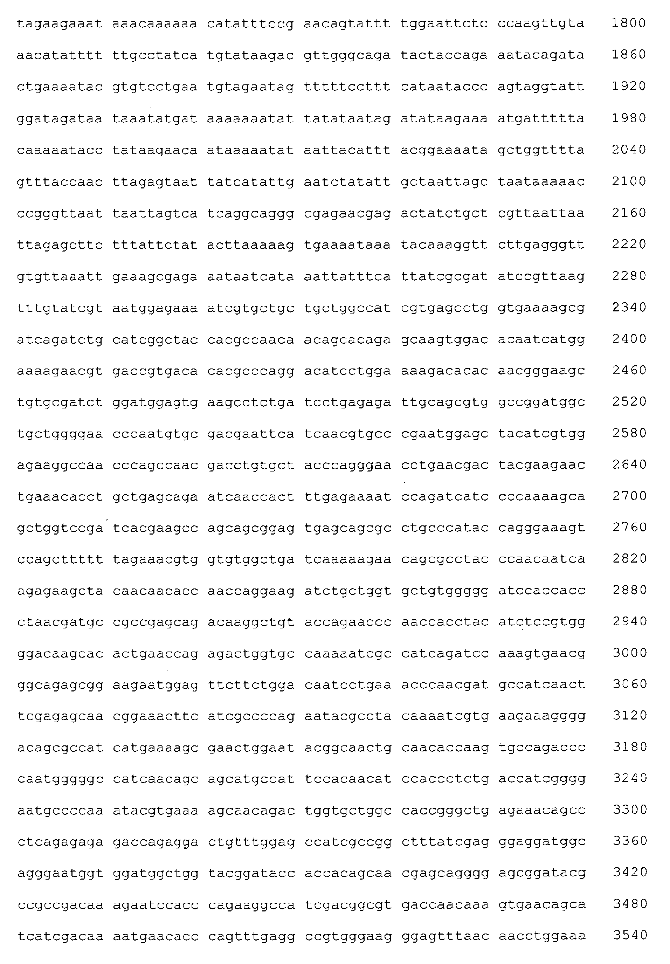

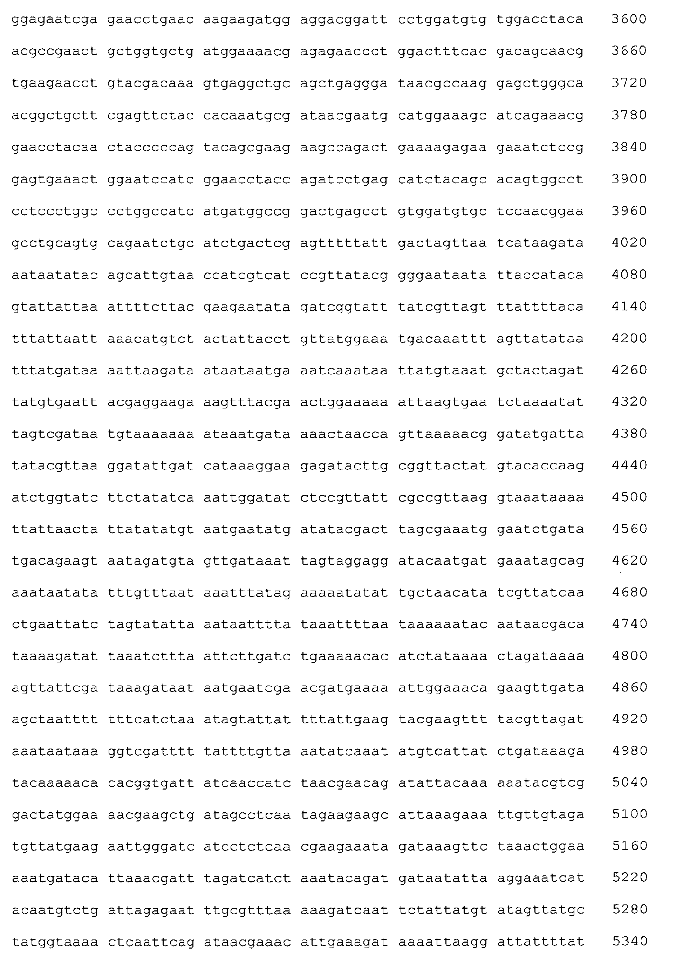

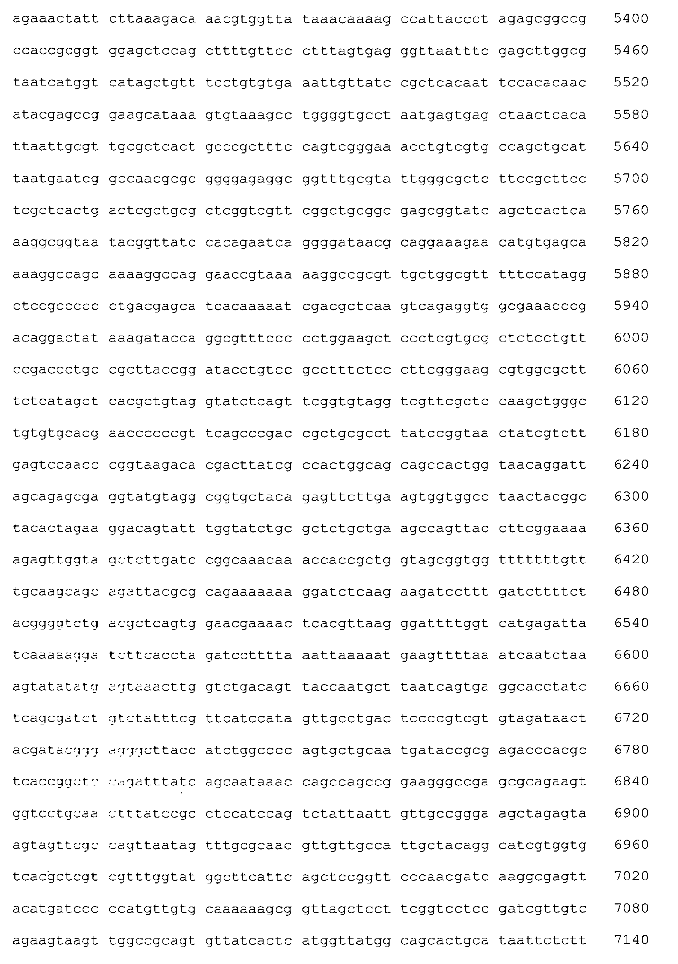

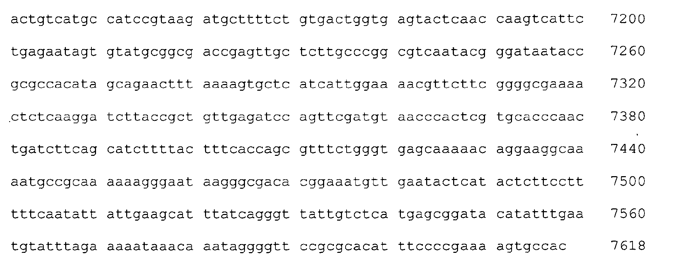

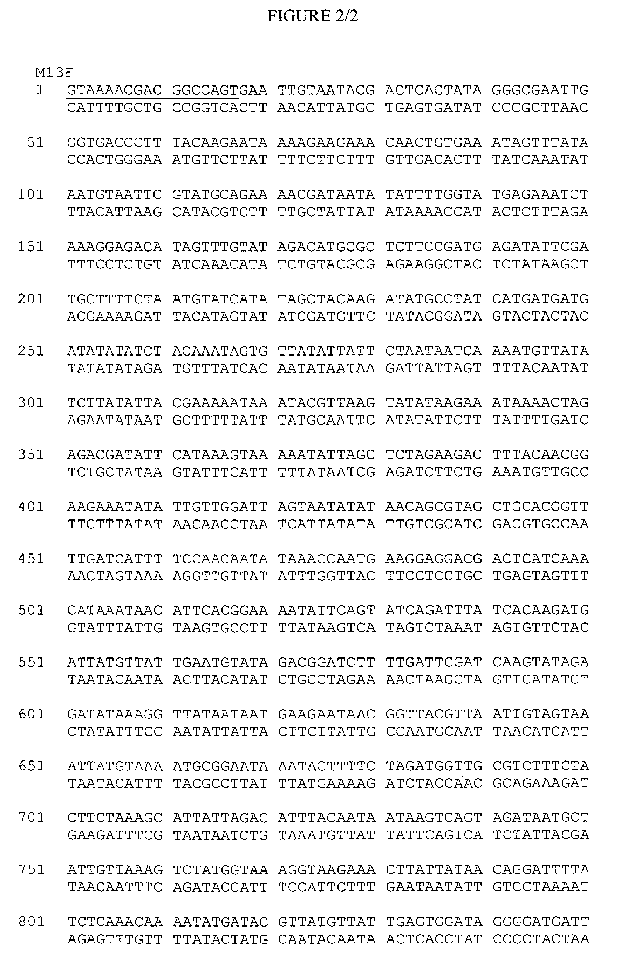

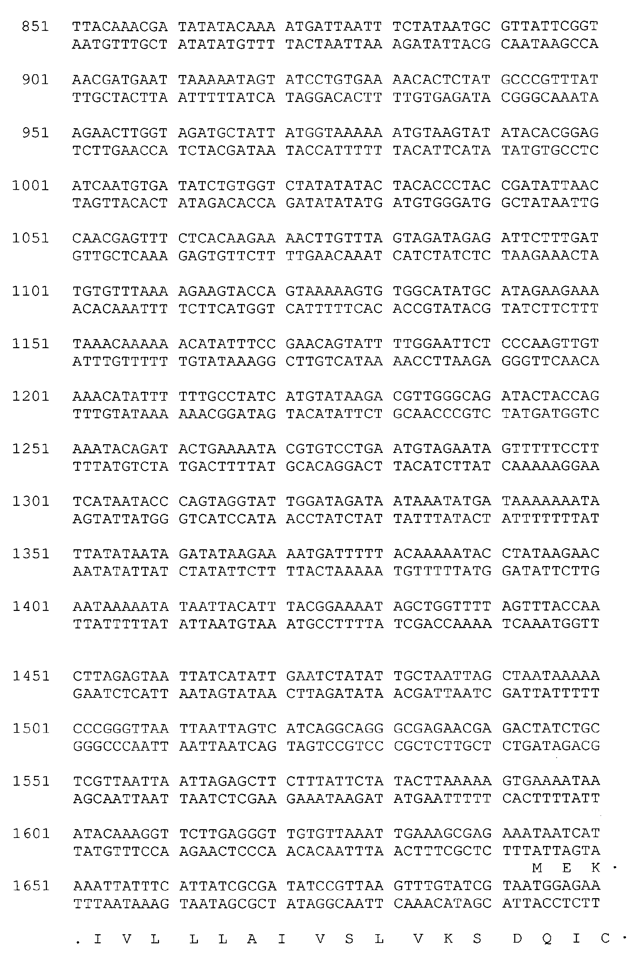

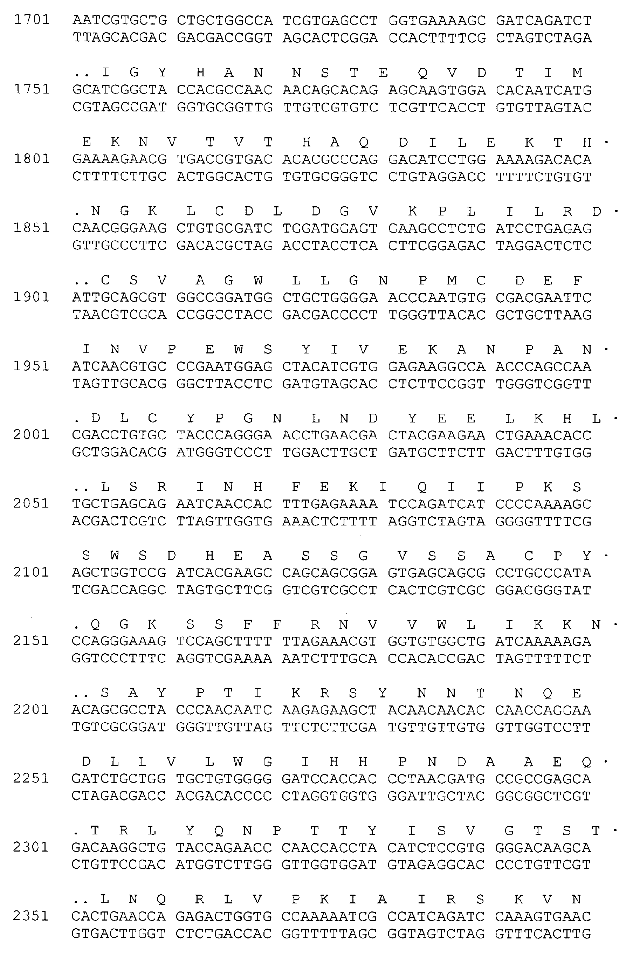

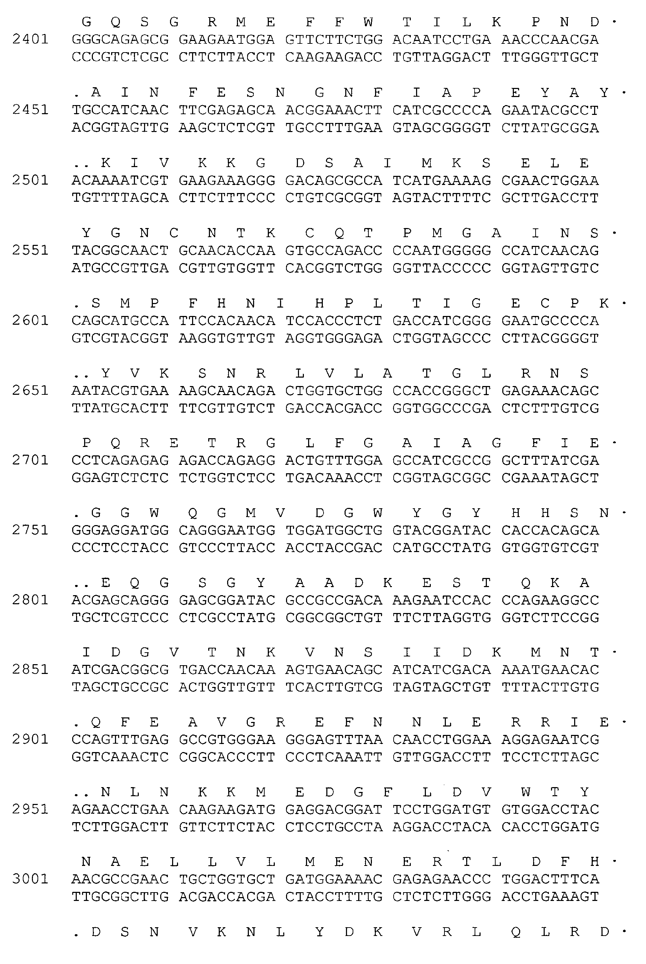

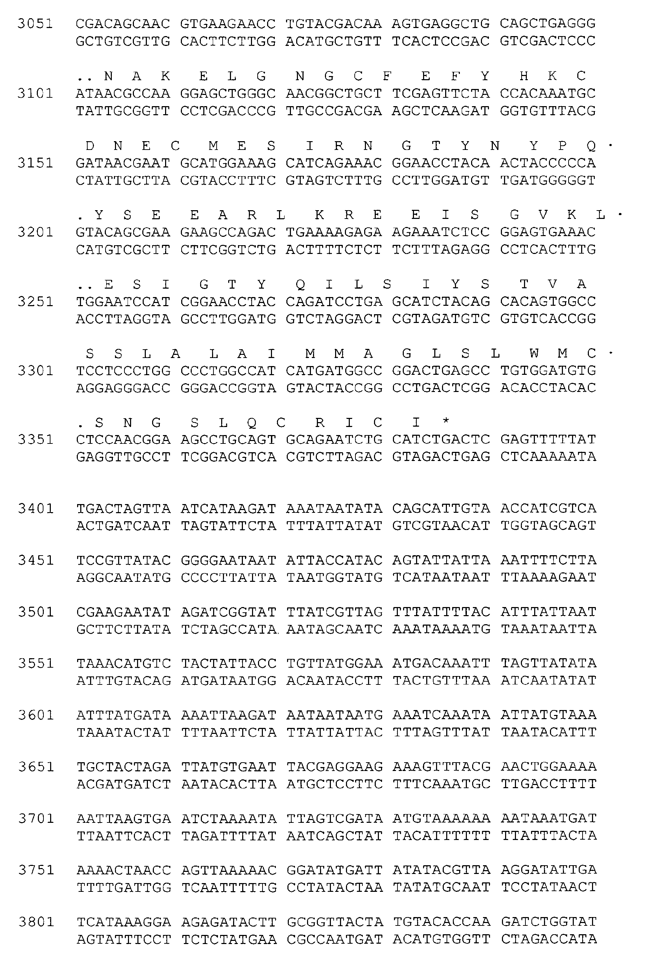

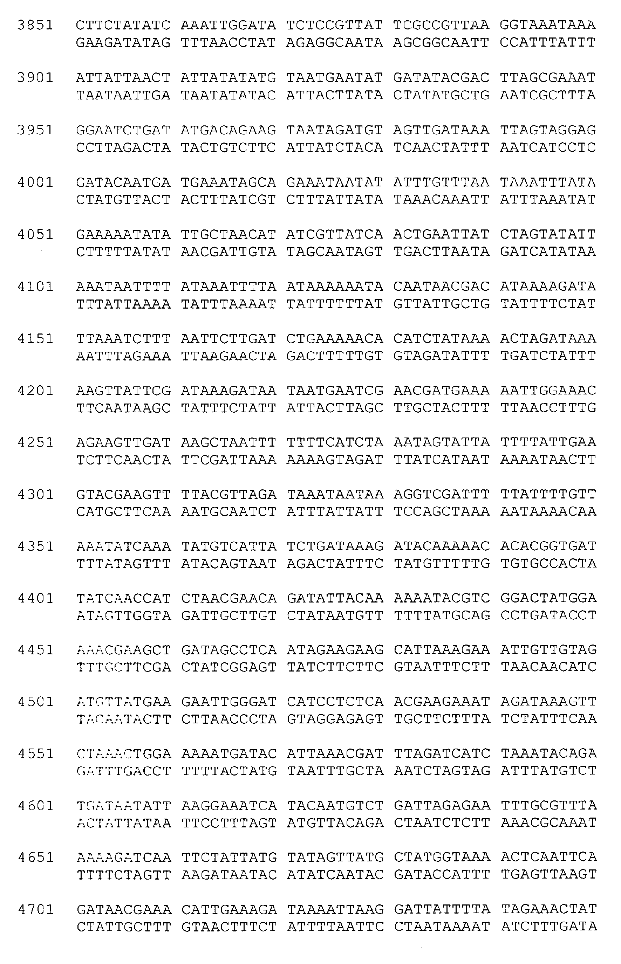

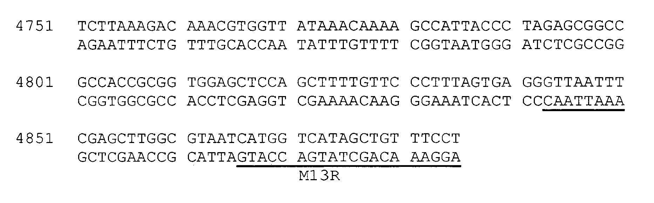

Dans un premier temps on a construit un plasmide donneur pJY1394.1 comprenant le gène synthétique HA modifié sous le contrôle du promoteur vaccinia H6 (Taylor J. et al., Vaccine, 1988, 6 : 504-508; Guo P. et al., J. Virol., 1989, 63 : 4189-4198; Perkus M. et al., J. Virol., 1989, 63 : 3829-3836.) et encadré par les bras flanquants du locus d'insertion F8 de manière à permettre l'insertion du gène HA dans le locus d'insertion F8 du génome du vecteur fowlpox. Le locus d'insertion F8 correspond au gène du fowlpox codant pour la photolyase décrit par Srinivasan et Tripathy (2005, Veterinary Microbiology 108 :215-223) ; ce gène est décrit également sous le nom de FPV158 dans la séquence complète du génome du fowlpox (GenBank, référence AF198100). L'insertion du gène HA et du promoteur H6 dans le locus F8 a pour conséquence la délétion du gène FPV158 du génome du virus fowlpox recombinant. La séquence nucléotidique complète du plasmide pJY1394.1 ainsi que la série de séquences nucléotidiques encadrant le gène HA (qui figurent également dans la séquence complète du plasmide) sont décrites respectivement dans les figures 1 et 2.

Le virus recombinant vFP2211 a ensuite été obtenu par double recombinaison entre les bras flanquants du plasmide pJY 1394.1 et le génome du fowlpox. Pour cela des cellules primaires d'embryon de poulets ont été transfectées avec le plasmide pJY1394.1 linéarisé par NotI et infectées (multiplicité d'infection de 10) avec la souche parentale fowlpox (vecteur TROVAC). Le vecteur TROVAC dérive de la souche vaccinale du vaccin Diftosec produit par Merial contre la variole du poulet. Les virus recombinants ont été sélectionnés par hybridation spécifiques sur plage de lyse avec une sonde détectant le gène HA inséré. Le vFP2211 ainsi isolé a ensuite été produit en flacons roulants sur cellules d'embryon de poulets.

### 1.1.2) Préparation du vaccin inactivé H5N9

Le vaccin inactivé H5N9 constitué de la souche faiblement pathogène H5N9 A/Chicken/Italy/22A/98 (fournie par le laboratoire d'Ilaria Capua (Istituto Zooprofilattico Sperimentale delle Venezie, Laboratorio Virologia, Padova, Italy)), qui a été produite sur oeufs embryonnés et inactivée à la beta-propiolactone (BPL), et d'une émulsion eau-dans-huile composée d'huile de paraffine, d'oleate de sorbitan et de polysorbate 80 a été préparé selon un procédé équivalent à celui décrit par Stone H. (1987, Avian Dis. 31: 483-490). L'HLB (Balance Lipohile Hydrophile) du mélange de tensio actifs de l'émulsion a une valeur de 5,3. Une dose vaccinale équivaut à 60 µl de liquide allantoidien titrant 10^{8.9} EID₅₀/ml avant inactivation.

### 1.1.3) Vaccination des poules pondeuses

Deux groupes (G1 et G2) de poussins de 1 jour (J0) de type Leghorn, ayant le statut « exempt d'organismes pathogènes spécifiques » (EOPS), ont été constitués. On a appliqué au groupe G1 le schéma de vaccination suivant : les poussins de 1 jour (J0) ont reçu par voie sous-cutanée, à la base du cou, à l'aide d'une seringue à insuline, 0,2 ml d'une suspension virale du vFP2211 titrant 3,0 log₁₀ DICC₅₀/0,2 ml. A l'âge de 8 semaines (S8), on a effectué le sexage des poulets. On a conservé environ 25 poulettes et 16 coqs que l'on a mis ensemble. Ils ont reçu à l'âge de 17 semaines (S17) une injection de rappel d'une dose vaccinale sous un volume de 0,5 ml du vaccin inactivé monovalent H5N9.

On a appliqué au groupe G2 le schéma de vaccination suivant : Les poussins EOPS de 3 semaines ont reçu par voie IM, dans le bréchet, à l'aide d'une seringue à insuline, une dose vaccinale sous un volume de 0,3 ml) du vaccin inactivé H5N9 monovalent. A l'âge de 8 semaines (S8), on a effectué le sexage des poulets. On a conservé environ 25 poulettes et 16 coqs que l'on a mis ensemble. Ils ont reçu à l'âge de 17 semaines (S17) une injection de rappel d'une dose vaccinale sous un volume de 0,5 ml) du vaccin inactivé monovalent H5N9.

### 1.1.4) Protocole d'infection des oeufs provenant des poules des groupes G1, G2 et de poules non vaccinées avec les souches de virus A/New Caledonia/20/99 IVR-116 (H1N1) ou A/New York/55/04 X-157 (H3N2).

On a évalué la capacité de réplication des souches vaccinales réassortantes A/New Caledonia/20/99 (xPR8) dénommée A/New Caledonia/20/99 IVR-116 (H1N1) et A/New York/55/04 (xPR8) dénommée A/New York/55/04 X-157 (H3N2) dans les oeufs des poules du groupe G1 qui ont été pondus à 40-41 (Test 2) et 49-50 (Test 3) semaines d'âge et dans les oeufs des poules du groupe G2 qui ont été pondus à 26-27 (Test 1), 40-41 (Test 2) et 49-50 (Test 3) semaines d'âge. En parallèle, on a évalué de la même façon la capacité de réplication des souches de virus A/New Caledonia/20/99 (xPR8) (H1N1) et A/New York/55/04 (xPR8) (H3N2) dans les oeufs témoins issus de poules EOPS non vaccinées (groupe témoin).

Tous les oeufs pondus pendant la semaine ont été conservés dans une enceinte à température contrôlée (12 à 15°C). Les oeufs d'un même test et provenant du même groupe d'animaux ont été rassemblés puis on a déclenché le processus d'embryogénèse en incubant les oeufs pendant 10 jours à 37-38°C dans une enceinte dont l'humidité relative était d'environ 70-80 %. Après 10 jours d'incubation, au moyen d'une mireuse, on a vérifié la vitalité de l'embryon dans chaque oeuf et on a repéré à l'aide d'une croix la cavité allantoïque. Les oeufs embryonnés d'un même test et provenant du même groupe d'animaux ont été rassemblés par pools de 8 oeufs. Les oeufs embryonnés d'un même pool ont été infectés avec la même dose infectieuse de virus grippal injecté sous un volume de 200 µl au niveau de la croix après avoir désinfecté puis percé la coquille de l'oeuf. Les doses infectieuses de 10², 10³ et 10⁴ EID₅₀ (Egg Infectious Dose 50 %) de virus A/New Caledonia/20/99 (xPR8) (H1N1) et A/New York/55/04 (xPR8) (H3N2) ont été testés. Les doses infectieuses de 10³et 10⁴ EID₅₀ par oeuf ont été testées dans les oeufs des tests 1 et 2. Les doses infectieuses de 10² et 10³ EID₅₀ par oeuf ont été testées dans les oeufs du test 3. Les oeufs embryonnés infectés ont ensuite été incubés à 34°C pendant 48 heures dans une enceinte dont l'humidité relative était de 80 % puis placés pendant une nuit à +4°C. On a prélevé ensuite le liquide allantoïque infecté de chaque oeuf. On a évalué le titre infectant sur chaque liquide allantoïque prélevé en mesurant le titre UHA (unités hémagglutinantes). Pour mesurer les titres UHA des liquides allantoïques infectés par la souche A/New York/55/04 (xPR8) (H3N2), on a utilisé des globules rouges de dinde. Pour mesurer les titres UHA des liquides allantoïques infectés par A/New Caledonia/20/99 (xPR8) (H1N1), on a utilisé des globules rouges de poule. Le titre UHA a été exprimé par l'inverse de la dernière dilution du liquide allantoïque infecté qui montrait une hémagglutination visible en présence d'une suspension de globules rouges de poule ou de dinde à 0,5 % en tampon phosphate.

### 1.2) Résultats

Les moyennes des titres UHA obtenus dans les oeufs provenant des différents groupes de poules vaccinées et non vaccinées en fonction de la dose infectieuse de virus injectée dans les oeufs et en fonction du moment de prélèvement des oeufs sont rassemblés dans les tableaux 1 et II

**Tableau I : Titres hémagglutinants obtenus dans les liquides allantoïques en fonction de la dose infectante de A/New Caledonia/20/99 (xPR8) (H1N1) utilisée, de la provenance des oeufs (G1, G2, témoin non vacciné) et du moment où les oeufs ont été pondus (test 1, test 2, test 3).**

| Dose infectante (EID₅₀/oeuf) | 100 | 1000 | | | 10000 | |
|---|---|---|---|---|---|---|
| Groupe | test 3 | test 1 | test 2 | test 3 | test 1 | test 2 |
| Témoin non vacciné | 640* | 1050 | 1660 | 1280 | 861 | 1522 |
| G1 (vFP2211/Vac. Inact. H5N9) | 1140 | NA | 1974 | 830 | NA | 1974 |
| G2 (Vac. Inact. H5N9/Vac. Inact. H5N9) | 806 | 905 | 1660 | 1159 | 905 | 1974 |

| | | | | | | |
|---|---|---|---|---|---|---|
| *: Titre hémagglutinant (UHA) exprimé sous la forme de moyenne géométrique des 8 titres UHA/50µl obtenus sur les liquides allantoïques des 8 oeufs inclus dans chaque condition. NA : Non applicable | | | | | | |

**Tableau II : Titres hémagglutinants obtenus dans les liquides allantoïques en fonction, de la dose infectante de A/New York/55/04 (xPR8) (H3N2) utilisée, de la provenance des oeufs (G1, G2, témoin non vacciné) et du moment où les oeufs ont été pondus (test 1, test 2, test 3).**

| Dose infectante (EID₅₀/oeuf) | 100 | 1000 | | 10000 |
|---|---|---|---|---|
| Groupe | test 3 | test 1 | test 3 | test 1 |
| Témoin non vacciné | 211* | 861 | 349 | 830 |
| G1 (vFP2211/Vac. inact. H5N9) | 88 | NA | 254 | NA |
| G2 (Vac. Inact. H5N9/Vac. Inact. H5N9) | 254 | 987 | 557 | 844 |

| | | | | |
|---|---|---|---|---|
| *:Titre hémagglutinant (UHA) exprimé sous la forme de moyenne géométrique des 8 titres UHA/50µl obtenus sur les liquides allantoïques des 8 oeufs inclus dans chaque condition. NA : Non applicable | | | | |

Les résultats obtenus dans les tableaux I et II montrent que les moyennes des titres UHA des liquides allantoïques des oeufs provenant de poules vaccinées (G1 et G2) sont équivalentes aux moyennes des titres UHA des liquides allantoïques des oeufs provenant de poules non vaccinées.

Une analyse statistique a été réalisée pour étudier les facteurs vaccination et dose. Les valeurs individuelles des UHA ont été transformées en log₂ puis analysées à l'aide d'un modèle de variance. L'hétérogénéité des variances a été testée à l'aide du test de l'étendue réduite, effectuée les résidus du modèle (logiciel SAS v8.2).

L'analyse statistique réalisée sur les valeurs individuelles des titres UHA a montré qu'il n'y avait pas d'effet vaccination. Il n'y avait pas non plus d'effet lié à la dose de virus qui a servi à l'infection des oeufs. Les titres UHA n'ont pas fluctué de façon sensible durant la période de ponte étudiée (allant de 26 semaines (test 1) à 50 semaines (test 3)).

### Exemple 2: Procédé de production de deux souches vaccinales pandémiques A/chicken/Italy/22A/98 (H5N9) et A/Vietnam/1194/04 NIBRG14 (H5N1) à partir d'oeufs embryonnés de poules qui ont été vaccinées, soit par deux injections d'un vaccin inactivé contenant une souche de virus grippal aviaire A/chicken/Italy/22A/98 (H5N9), soit par une injection d'un avipoxvirus recombinant codant pour l'HA d'une souche H5N1 suivi d'une deuxième injection d'un vaccin inactivé H5N9 contenant une souche de virus grippal aviaire.

### 2.1.1) Vaccination des poules

Le protocole de vaccination des poules a été identique à celui qui a été utilisé dans l'exemple 1.

### 2.1.2) Protocole d'infection des oeufs provenant des poules des groupes G1, G2 et de poules non vaccinées avec les souches de virus A/chicken/Italy/22A/98 (H5N9) et A/Vietnam/1194/04 NIBRG14 (H5N1).

Le protocole d'infection a été le même que celui qui a été utilisé dans l'exemple 1 hormis les modifications suivantes :
On a utilisé la souche aviaire faiblement pathogène A/chicken/Italy/22A/98 (H5N9) provenant du Laboratoire Istituto Zooprofilattico Sperimentale delle Venezie, Laboratorio Virologia, Padova, Italy et une souche vaccinale réassortante pandémique atténuée A/Vietnam/1194/04 NIBRG14 (H5N1) pour infecter les oeufs. Cette dernière souche a été fournie par le laboratoire National Institute for Biological Standards and Control (NIBSC) South Mimms, Potters Bar, Herts EN6 3QG, UK et a été obtenue par génétique inverse, comme cela est décrit par Nicolson C. et coll., Génération of influenza vaccine viruses on Vero cells by reverse genetics: an H5N1 candidate vaccine strain produced under a quality system, 2005, Vaccine, 23:2943-2952 .

Les doses infectieuses de 10², 10³, 10⁴ et 10⁵ EID₅₀ (Egg Infectious Dose 50 %) ont été testées pour ces deux souches. Les doses infectieuses de 10³, 10⁴ et 10⁵ EID₅₀ par oeuf ont été testées dans les oeufs du test 1. Les doses infectieuses de 10⁴ et 10⁵ EID₅₀ par oeuf ont été testées dans les oeufs du test 2. Les doses infectieuses de 10² et 10³ EID₅₀ par oeuf ont été testées dans les oeufs du test 3.

Pour mesurer les titres UHA des liquides allantoïques infectés par A/chicken/Italy/22A/98 (H5N9) ou A/Vietnam/1194/04 NIBRG14 (H5N1), on a utilisé les globules rouges de poule.

### 2.2) Résultats

Les titres UHA obtenus dans les oeufs provenant des différents groupes de poules vaccinées et non vaccinées en fonction de la dose infectieuse de virus injectée dans les oeufs et en fonction du moment de prélèvement des oeufs sont rassemblés dans les tableaux III et IV

**Tableau III : Titres hémagglutinants obtenus dans les liquides allantoïques en fonction de la dose infectante de A/chicken/Italy/22A/98 (H5N9) utilisée, de la provenance des oeufs (G1, G2, témoin non vacciné) et du moment où les oeufs ont été pondus (test 1, test 2, test 3).**

| Dose infectante (EID₅₀/oeuf) | 100 | 1000 | | 10000 | | 100000 | |
|---|---|---|---|---|---|---|---|
| Groupe | test 3 | test 1 | test 3 | test 1 | test 2 | test 1 | test 2 |
| Témoin non vacciné | 35* | 53 | 32 | 53 | 64 | 54 | 53 |
| G1 (vFP2211/Vac. Inact. H5N9) | 64 | NA | 54 | NA | 59 | NA | 86 |
| G2 (Vac. Inact. H5N9/Vac. Inact. H5N9) | 102 | 64 | 57 | 59 | 70 | 102 | 59 |

| | | | | | | | |
|---|---|---|---|---|---|---|---|
| *: Titre hémagglutinant (UHA) exprimé sous la forme de moyenne géométrique des 8 titres UHA/50 µl obtenus sur les liquides allantoïques des 8 oeufs inclus dans chaque condition. NA : Non applicable | | | | | | | |

**Tableau IV: Titres hémagglutinants obtenus dans les liquides allantoïques en fonction de la dose infectante de A/Vietnam/1194/04 (H5N1) RG14 utilisée, de la provenance des oeufs (G1, G2, témoin non vacciné) et du moment où les oeufs ont été pondus (test 1, test 2, test 3).**

| Dose infectante (EID₅₀/oeuf) | 1000 | 10000 | | 100000 | |
|---|---|---|---|---|---|
| groupe | test 1 | test 1 | test 2 | test 1 | test 2 |
| Témoin non vacciné | 194* | 232 | 197 | 172 | 181 |
| G1 (vFP2211/Vac. Inact. H5N9) | NA | NA | 197 | NA | 166 |
| G2 (Vac. Inact. H5N9/Vac. Inact. H5N9) | 152 | 279 | 215 | 181 | 235 |

| | | | | | |
|---|---|---|---|---|---|
| *: Titre hémagglutinant (UHA) exprimé sous la forme de moyenne géométrique des 8 titres UHA/50 µl obtenus sur les liquides allantoïques des 8 oeufs inclus dans chaque condition. NA : Non applicable | | | | | |

Les résultats présentés dans les tableaux III et IV ainsi que l'analyse statistique montrent que les titres UHA des liquides allantoïques provenant des oeufs des poules vaccinées (G1 et G2) sont similaires aux titres UHA des liquides allantoïques des oeufs des poules non vaccinées, même dans la situation où les poules ont été vaccinées avec une souche identique à celle qui a été utilisée pour infecter les oeufs embryonnés provenant de ces poules (cas des groupes G1 et G2 immunisés avec la souche A/chicken/Italy/22A/98 (H5N9). Il n'y a pas non plus d'effet lié à la dose de virus qui sert à l'infection des oeufs. Les titres UHA n'ont pas fluctué de façon sensible durant la période de ponte étudiée (allant de 26 semaines (test 1) à 50 semaines (test 3)).

L'analyse statistique a été réalisée comme dans l'exemple 1.

### Exemple 3 : Analyse de la réponse en anticorps maternels anti-H5 dans les oeufs de poules qui ont été vaccinées avec la souche vaccinale A/chicken/Italy/22A/98 (H5N9)

### 3.1) protocole opératoire

On a mesuré la présence d'anticorps anti H5 dans les oeufs des poules des groupes G1 et G2 qui ont été vaccinées avec la souche A/chicken/Italy/22A/98 (H5N9) selon le protocole décrit dans le paragraphe 1.1.1). En parallèle, on a également analysé la réponse anti H5 dans les jaunes d'oeufs de poules non vaccinées (groupe témoin).

On a analysé la réponse anti H5 dans les jaunes d'oeufs (ou liquides vitellins) qui ont été prélevés au moment des tests 1 (S26-27), 2 (S40-41) et 3 (S49-50). L'analyse de la réponse anti-H5 dans les jaunes d'oeufs du test 1 a été faite avant embryogénèse (J0) et après embryogénèse (J10) (c'est-à-dire après la phase d'incubation de 10 jours à 37°C). L'analyse des réponses anti H5 dans les jaunes d'oeufs des tests 2 et 3 a été faite uniquement après embryogénèse (J10). La même analyse a été également faite avec des oeufs provenant de poules non vaccinées (Voir paragraphe 3.2.2). On a également étudié la réponse anti H5 dans les liquides allantoïque des oeufs du test 2 après embryogénèse (J10). Les jaunes d'oeufs ont été prélevés par aspiration au moyen d'une pipette à embout jetable. A l'exception du test 1, où les jaunes d'oeufs ont été gardés congelés sans avoir été préalablement dilués, les jaunes d'oeufs des tests 2 et 3, et les liquides allantoïques ont été préalablement dilués au 1/5^{ème} en tampon phosphate avant congélation. Les jaunes d'oeufs dilués et les liquides allantoïques ont été conservés congelés jusqu'au moment du dosage des anticorps anti H5 qui a été fait en utilisant la méthode de dosage par inhibition d'hémagglutination (IHA) des globules rouges de poules qui se produit en présence de la souche A/chicken/Italy/22A/98 (H5N9). Le dosage était basé sur la capacité des anticorps neutralisants dirigés spécifiquement contre la HA du virus à inhiber l'activité « hémagglutinante » du virus. Dans ce test, on a utilisé du sérum de mouton anti A/Vietnam/1194/04 fourni par le NISBC comme témoin positif et du sérum de souris naïve comme témoin négatif On a réalisé des dilutions successives de raison 2 des prélèvements (jaunes d'oeufs dilués ou liquides allantoïques) dans une microplaque à fond conique afin d'obtenir 50 µl de chacune des dilutions par puits. On a ajouté dans chaque puits 50 µl d'une suspension virale titrant 4 unités hémagglutinantes (4HAU) et provenant d'un liquide allantoïque clarifié qui a été infecté par la souche A/chicken/Italy/22A/98 (H5N9) fournie par le laboratoire d'Ilaria Capua (Istituto Zooprofilattico Sperimentale delle Venezie, Laboratorio Virologia, Padova, Italy). On a laissé incuber pendant 1 heure à la température du laboratoire avant d'ajouter 50 µl d'une solution de globules rouges de poule à 0,5 % en tampon phosphate. On a laissé reposer pendant 1 heure à +4°C avant d'effectuer la lecture du test. La présence d'une inhibition d'hémagglutination se traduisait par la présence d'un point rouge au fond du micropuits tandis que la présence d'une hémagglutination se traduisait par la présence d'un halo rosé dans le micropuits. Le titre en anticorps IHA était représenté par l'inverse de la dernière dilution où l'on n'observe pas d'hémagglutination dans le micropuits.

### 3.2) Résultats

### 3.2.1) analyse de la réponse anti-H5 dans les jaunes d'oeufs du test 1

A J0, 8 des 8 jaunes d'oeufs analysés du groupe non vacciné étaient négatifs dans le test IHA tandis que 6 des 8 jaunes d'oeufs provenant du groupe 2 étaient positifs dans le test IHA ce qui indiquait la présence d'anticorps anti H5.

A J10, 4 des 4 jaunes d'oeufs analysés du groupe non vacciné étaient négatifs dans le test IHA tandis que 4 des 4 jaunes d'oeufs provenant du groupe 2 étaient positifs dans le test IHA.

### 3.2.2) analyse de la réponse anti-H5 dans les jaunes d'oeufs et les liquides allantoïques du test 2.

A J10, 5 des 5 jaunes d'oeufs analysés du groupe non vacciné étaient négatifs dans le test IHA tandis que 4 des 5 jaunes d'oeufs provenant du groupe 1, et 4 des 5 jaunes d'oeufs provenant du groupe 2 étaient positifs dans le test IHA ce qui indiquait l'existence d'anticorps anti H5 dans ces groupes. Les valeurs individuelles des titres IHA obtenus sont rassemblées dans le tableau V.

**Tableau V : Titres IHA des jaunes d'oeufs prélevés après embryogénèse (J10)**

| Jaunes d'oeufs | J10* |
|---|---|
| | 160 |
| Groupe 1 | 80 |
| (vFR2211/Vac. Inact. H5N9) | 320 |
| | 80 |
| | <5 |
| | 80 |
| Groupe 2 | 160 |
| (Vac. Inact. H5N9/Vac. Inact. H5N9) | 40 |
| | 40 |
| | <5 |
| | <5 |
| Groupe Non Vacciné | <5 |
| | <5 |
| | <5 |
| | <5 |

| | |
|---|---|
| *: les titres à J10 proviennent de jaunes d'oeufs différents | |

Par contre tous les liquides allantoïques provenant des groupes 1 et 2 étaient négatifs dans le test IHA, ce qui signifie qu'il n'y avait pas d'anticorps inhibant l'hémagglutination du virus grippal H5N9 détectables dans les liquides allantoïques.

### 3.2.3) analyse de la réponse anti-H5 dans les jaunes d'oeufs du test 3

A J10, 5 des 5 jaunes d'oeufs analysés du groupe non vacciné étaient négatifs dans le test IHA tandis que 5 des 5 jaunes d'oeufs provenant du groupe 1 étaient positifs dans le test IHA.

Les valeurs individuelles des titres IHA sont rassemblées dans le tableau VI.

**Tableau VI : Titres IHA des jaunes d'oeufs prélevés après embryogénèse (J10) en fonction de la provenance des oeufs**

| Jaunes d'oeufs | J10 |
|---|---|
| | 80 |
| Groupe 1 | 80 |
| (vFP2211/Vac. Inact. H5N9) | 160 |
| | 5 |
| | 320 |
| | <5 |
| | <5 |
| Groupe non vacciné | <5 |
| | <5 |
| | <5 |

En conclusion, sur la base des résultats des exemples 2 et 3, la présence d'anticorps maternels anti H5N9 qui a été révélée dans les jaunes d'oeufs des 3 tests est sans incidence sur la productivité virale des liquides allantoïques infectés par une souche H5N1 ou H5N9.

### Exemple 4 : Analyse de la réponse sérologique des poules vaccinées avec la souche vaccinale A/chicken/Italy/22A/98 (H5N9) et des anticorps maternels présents anti-H5 dans les oeufs de ces poules vaccinées

Des prélèvements sanguins ont été effectués à 28 et 36 semaines d'âge chez les poules vaccinées des groupes 1 et 2. Les oeufs pondus par ces poules ont été récoltés aux semaines 27 et 37 pour évaluer l'importance du transfert des anticorps maternels dans les jaunes d'oeufs. La réponse anticorps anti -H5 a été évaluée au moyen du test d'inhibition de l'hémagglutination en utilisant comme antigène la souche H5N9 A/chicken/Italy/22A/98 homologue à la souche du vaccin inactivé. Les résultats sont exprimés en log₁₀ au tableau VII.

**Tableau VII: Taux d'anticorps inhibant l'hémagglutination (antigène H5N9 A/chicken/Italy/22A/98) dans le sérum des poules pondeuses vaccinées et dans le vitellus des oeufs pondus par ces poules**

| | Sérum | Vitellus | Sérum | Vitellus |
|---|---|---|---|---|
| Semaine d'âge | 28 | 27 | 36 | 37 |
| G1 | 2,10±0,61 (15)* | 1,96±0,94 (21)** | 1,92±0,91 (15) | 1,63±0,89 (10) |
| G2 | 2,20±0,62 (15) | 1,81±0,88 (13) | 2,26±0,65 (15) | 1,43±0,89 (14) |

| | | | | |
|---|---|---|---|---|
| * moyenne géométrique des titres en anticorps inhibant l'hémagglutination exprimé en log₁₀ ± écart type (nombre d'échantillons testés par groupe) ** la dilution la plus faible testée sur les vitellus est de 1/10 (1 log₁₀) ; pour le calcul des moyennes et écart type, les valeurs des oeufs négatifs à la dilution 1/10 ont été mises à 0,7 log₁₀. | | | | |

**Tableau VIII: Taux d'anticorps inhibant l'hémagglutination (antigène H5N9 A/turkey/Wisconsin/68) dans le vitellus des oeufs pondus par les poules vaccinées des groupes 1 et 2.**

| | Vitellus |
|---|---|
| Semaine d'âge | 27 |
| G1 | 1,67±0,79 (21)* |
| G2 | 1,39±0,80 (13) |

| | |
|---|---|
| * moyenne géométrique des titres en anticorps inhibant l'hémagglutination exprimé en log₁₀ ± déviation standard (nombre d'échantillons testés par groupe) ; la dilution la plus faible testée est de 1/10 (1 log₁₀) ; pour le calcul des moyennes et écart type, les valeurs des oeufs négatifs à la dilution 1/10 ont été mises à 0,7 log₁₀. | |

Ces résultats confirment la transmission des anticorps maternels dans le jaune d'oeufs des oeufs pondus par les poules vaccinées des groupes 1 et 2.

Les taux d'anticorps anti-H5N9 homologues (tableau VII) dans le vitellus sont supérieurs aux titres anti-H5N9 hétérologues (tableau VIII) bien que cette différence ne soit pas statistiquement significative.

### Exemple 5 : Production de la souche H5N9 A/turkey/Wisconsin/68 sur des oeufs pondus par les poules vaccinées par un vaccin inactivé H5N9 (souche A/chicken/Italy/22A/98) du groupe 2

### 5.1. Protocole opératoire

Les oeufs (environ 220-230) des poules du groupe 2 vaccinées 2 fois à 3 et 17 semaines d'âge avec le vaccin inactivé H5N9 (souche A/chicken/Italy/22A/98) ont été prélevés pendant les semaines 28 et 29. Ces oeufs, après stockage à température contrôlée (entre 12 et 15°C), ont été incubés à 37°C pendant 11 jours. Après mirage pour vérifier la bonne viabilité des embryons, les oeufs ont été inoculés dans la cavité allantoïque avec 0.2 ml d'une dilution d'une solution virale stock de la souche_H5N9 A/turkey/Wisconsin/68 titrant 9,68 log₁₀ EID₅₀/ml (inoculum). Sept groupes d'environ 30 oeufs ont été formés. Quatre groupes (G1 à G4) ont été inoculés avec les dilutions suivantes de l'inoculum: 10⁻⁵, 10⁻⁴, 10⁻³ et 10⁻². Trois autres groupes (G5 à G7) ont été inoculés avec la dilution 10⁻³ de l'inoculum. Après inoculation, les oeufs ont été incubés à 37°C±1,5°C sous 70 %±10 % d'humidité. Après 20 h d'incubation, les oeufs ont été mirés pour éliminer les oeufs morts issus de l'inoculation et ils ont été ensuite remis en incubation dans les mêmes conditions. Quarante deux heures après l'inoculation, les oeufs ont été mis au froid avant récolte du liquide allantoïque. Les liquides allantoïques des oeufs des groupes 1 à 4 ont été rassemblés (un pool de liquide par groupe). Les liquides allantoïques des 4 groupes d'oeufs ont été stockés à -70°C avant titrage en unité hémagglutinante et en dose infectieuse 50 % pour l'oeuf (EID₅₀).

### 5.2. Résultats

Les résultats sont rapportés au tableau IX.

**Tableau IX : Titres hémagglutinants et infectieux obtenus après production de la souche H5N9 A/turkey/Wisconsin/68 sur des oeufs provenant de poules vaccinées avec un vaccin inactivé contenant la souche H5N9 A/chicken/Italy/22A/98.**

| Groupe | Dilution de l'inoculum | Titre en unité hémagglutinante (log₁₀) | Titre infectieux EID₅₀ (log₁₀/ml) |
|---|---|---|---|
| 1 | 10⁻⁵ | 2,25 | 9,50 |
| 2 | 10⁻⁴ | 2,40 | 9,33 |
| 3 | 10⁻³ | 2,25 | 9,67 |
| 4 | 10⁻² | 2,40 | 9,88 |

Ces résultats montrent des titres très similaires entre les groupes et il n'y a donc pas d'effet de la dilution de l'inoculum. De plus, les titres obtenus sont très élevés et sont comparables à ceux obtenus sur oeufs ne possédant pas d'anticorps maternels anti-H5N9 (données historiques du laboratoire). Ces résultats confirment donc que, dans les conditions testées, la présence d'anticorps maternels dans les oeufs n'interfère pas avec la production d'un virus grippal de même sous-type.

## Revendications

1. Un procédé de production du virus de la grippe selon lequel :
a. On administre à des poules un vaccin contre la grippe,
b. on prélève les oeufs des poules vaccinées,
c. on déclenche le processus d'embryogénèse,
d. on infecte les oeufs embryonnés en inoculant un virus de la grippe dans la cavité allantoïque,
e. on incube les oeufs embryonnés infectés dans des conditions de température et d'humidité qui permettent la réplication du virus et,
f. on récolte le liquide allantoïque contenant le virus.

2. Un procédé selon la revendication 1, selon lequel le vaccin protège les poules contre la grippe aviaire.

3. Un procédé selon la revendication 1 ou 2, selon lequel le vaccin contre la grippe comprend dans sa composition l'hémagglutinine d'un virus de la grippe sous la forme de protéine et/ou de gène codant pour cette protéine.

4. Un procédé selon l'une des revendications 1 à 3, selon lequel la composition du vaccin contre la grippe contient un virus grippal entier et inactivé.

5. Un procédé selon l'une des revendications 1 à 4, selon lequel la composition du vaccin contre la grippe contient un produit dérivé d'un virus grippal entier.

6. Un procédé selon l'une des revendications 1 à 5, selon lequel la composition du vaccin contient en outre un adjuvant.

7. Un procédé selon l'une des revendications 1 à 3, selon lequel la composition du vaccin contre la grippe contient un virus grippal atténué.

8. Un procédé selon l'une des revendications 1 à 3, selon lequel le vaccin contre la grippe comprend un vecteur comprenant un gène codant pour l'hémagglutinine d'un virus grippal.

9. Un procédé selon la revendication 8, selon lequel ce vecteur est un poxvirus.

10. Un procédé selon la revendication 8 ou 9, selon lequel le vecteur comprend également un gène codant pour la neuraminidase d'un virus grippal.

11. Un procédé selon l'une des revendications 8 à 10, selon lequel la composition du vaccin contient en outre un adjuvant.

12. Un procédé selon l'une des revendications 3 à 11, selon lequel l'hémagglutinine du virus de la grippe sous la forme de protéine et/ou de gène codant pour cette protéine contenue dans la composition du vaccin qui est administré aux poules et l'hémagglutinine du virus de la grippe qui est utilisé pour infecter la cavité allantoïque des oeufs embryonnés des poules vaccinées ont des sous types différents.

13. Un procédé selon l'une des revendications 3 à 11, selon lequel l'hémagglutinine du virus de la grippe sous la forme de protéine et/ou de gène codant pour cette protéine contenue dans la composition du vaccin qui est administré aux poules et l'hémagglutinine du virus de la grippe qui est utilisé pour infecter la cavité allantoïque des oeufs embryonnés des poules vaccinées ont le même sous type.

14. Un procédé selon l'une des revendications 3 à 11, selon lequel l'hémagglutinine du virus de la grippe sous la forme de protéine et/ou de gène codant pour cette protéine contenue dans la composition du vaccin qui est administré aux poules et l'hémagglutinine du virus de la grippe qui est utilisé pour infecter la cavité allantoïque des oeufs embryonnés des poules vaccinées sont identiques.

15. Un procédé selon l'une des revendications 4, 6 ou 7, selon lequel le virus de la grippe contenu dans la composition du vaccin qui est administré aux poules est identique au virus de la grippe qui est utilisé pour infecter la cavité allantoïque des oeufs embryonnés des poules vaccinées.

16. Un procédé selon l'une des revendications 12 à 15, selon lequel l'hémagglutinine du virus de la grippe sous la forme de protéine et/ou de gène codant pour cette protéine contenue dans la composition du vaccin qui est administré aux poules et l'hémagglutinine du virus de la grippe qui est utilisé pour infecter la cavité allantoïque des oeufs embryonnés des poules vaccinées ont le sous type H5, H6, H7 ou H9.

17. Un procédé selon la revendication 16, selon lequel l'hémagglutinine du virus de la grippe sous la forme de protéine et/ou de gène codant pour cette protéine contenue dans la composition du vaccin qui est utilisé pour vacciner les poules contre la grippe et l'hémagglutinine du virus de la grippe qui est utilisé pour infecter la cavité allantoïque des oeufs embryonnés des poules vaccinées ont le sous type H5 ou H7.

18. Un procédé selon l'une des revendications 1 à 17, comprenant une étape supplémentaire de purification du virus.

19. Un procédé selon l'une des revendications 1 à 18, comprenant une étape supplémentaire d'inactivation du virus.

20. Utilisation d'un procédé selon l'une des revendications 1 à 19, pour la fabrication d'un vaccin destiné à la prévention de la grippe.

21. Utilisation d'un procédé selon l'une des revendications 1 à 19, pour la fabrication d'un vaccin destiné à la prévention de la grippe humaine pandémique.

22. Utilisation d'un procédé selon l'une des revendications 1 à 19, pour la fabrication d'un vaccin destiné à la prévention de la grippe humaine épidémique.

23. Utilisation d'un procédé selon l'une des revendications 1 à 19, pour la fabrication d'un vaccin destiné à la prévention de la grippe chez les équidés, les suidés, les canidés, les félidés, les mustélidés et les espèces aviaires.

24. Utilisation des oeufs de poules vaccinées contre la grippe pour la production d'un virus grippal.

25. Utilisation des oeufs de poules vaccinées contre la grippe pour la fabrication d'un vaccin contre la grippe.

26. Utilisation des oeufs de poules vaccinées contre la grippe selon la revendication 24 ou 25, selon laquelle les oeufs contiennent des anticorps dirigés contre l'hémagglutinine du virus de la grippe.

27. Utilisation selon la revendication 26, selon laquelle les anticorps sont dirigés contre l'hémagglutinine du virus de la grippe ayant le sous type H5, H6, H7 ou H9.

## Patentansprüche

1. Verfahren zur Produktion von Grippevirus, bei dem man
a. Hühnern einen Grippeimpfstoff verabreicht,
b. die Eier der geimpften Hühner entnimmt,
c. den Embryogenesevorgang auslöst,
d. die embryonieren Eier durch Inokulieren eines Grippevirus in die Allantoishöhle infiziert,
e. die infizierten embryonierten Eier unter Temperatur- und Feuchtigkeitsbedingungen, die die Virusreplikation gestatten, inkubiert und
f. die Allantoisflüssigkeit, die das Virus enthält, gewinnt.

2. Verfahren nach Anspruch 1, wobei der Impfstoff die Hühner gegen die Vogelgrippe schützt.

3. Verfahren nach Anspruch 1 oder 2, wobei der Grippeimpfstoff in seiner Zusammensetzung das Hämagglutinin eines Grippevirus in Proteinform und/oder in Form des Gens, das für das Protein codiert, umfasst.

4. Verfahren nach einem der Ansprüche 1 bis 3, wobei die Grippeimpfstoffzusammensetzung ein ganzes und inaktiviertes Grippevirus enthält.

5. Verfahren nach einem der Ansprüche 1 bis 4, wobei die Grippeimpfstoffzusammensetzung ein Produkt, das von einem ganzen Grippevirus stammt, enthält.

6. Verfahren nach einem der Ansprüche 1 bis 5, wobei die Impfstoffzusammensetzung weiterhin ein Adjuvans enthält.

7. Verfahren nach einem der Ansprüche 1 bis 3, wobei die Grippeimpfstoffzusammensetzung attenuiertes Grippevirus enthält.

8. Verfahren nach einem der Ansprüche 1 bis 3, wobei der Grippeimpfstoff einen Vektor umfasst, der ein Gen umfasst, das für das Hämagglutinin eines Grippevirus codiert.

9. Verfahren nach Anspruch 8, wobei es sich bei dem Vektor um ein Poxvirus handelt.

10. Verfahren nach Anspruch 8 oder 9, wobei der Vektor auch ein Gen, das für die Neuraminidase eines Grippevirus codiert, umfasst.

11. Verfahren nach einem der Ansprüche 8 bis 10, wobei die Impfstoffzusammensetzung weiterhin ein Adjuvans enthält.

12. Verfahren nach einem der Ansprüche 3 bis 11, wobei das Hämagglutinin eines Grippevirus in Proteinform und/oder in Form des Gens, das für das Protein codiert, das in der Impfstoffzusammensetzung, die an die Hühner verabreicht wird, enthalten ist und das Hämagglutinin des Grippevirus, mit dem die Allantoishöhle von embryonierten Eiern von geimpften Hühnern infiziert werden, verschiedene Subtypen aufweisen.

13. Verfahren nach einem der Ansprüche 3 bis 11, wobei das Hämagglutinin eines Grippevirus in Proteinform und/oder in Form des Gens, das für das Protein codiert, das in der Impfstoffzusammensetzung, die an die Hühner verabreicht wird, enthalten ist und das Hämagglutinin des Grippevirus, mit dem die Allantoishöhle von embryonierten Eiern von geimpften Hühnern infiziert werden, denselben Subtyp aufweisen.

14. Verfahren nach einem der Ansprüche 3 bis 11, wobei das Hämagglutinin eines Grippevirus in Proteinform und/oder in Form des Gens, das für das Protein codiert, das in der Impfstoffzusammensetzung, die an die Hühner verabreicht wird, enthalten ist und das Hämagglutinin des Grippevirus, mit dem die Allantoishöhle von embryonierten Eiern von geimpften Hühnern infiziert werden, identisch sind.

15. Verfahren nach einem der Ansprüche 4, 6 oder 7, wobei das Grippevirus, das in der Impfstoffzusammensetzung, die den Hühnern verabreicht wird, enthalten ist, mit dem Grippevirus, mit dem die Allantoishöhle von embryonierten Eiern von geimpften Hühnern infiziert werden, identisch ist.

16. Verfahren nach einem der Ansprüche 12 bis 15, wobei das Hämagglutinin eines Grippevirus in Proteinform und/oder in Form des Gens, das für das Protein codiert, das in der Impfstoffzusammensetzung, die an die Hühner verabreicht wird, enthalten ist und das Hämagglutinin des Grippevirus, mit dem die Allantoishöhle von embryonierten Eiern von geimpften Hühnern infiziert werden, den Subtyp H5, H6, H7 oder H9 aufweisen.

17. Verfahren nach Anspruch 16, wobei das Hämagglutinin eines Grippevirus in Proteinform und/oder in Form des Gens, das für das Protein codiert, das in der Impfstoffzusammensetzung, die an die Hühner gegen die Grippe verabreicht wird, enthalten ist und das Hämagglutinin des Grippevirus, mit dem die Allantoishöhle von embryonierten Eiern von geimpften Hühnern infiziert werden, den Subtyp H5 oder H7 aufweisen.

18. Verfahren nach einem der Ansprüche 1 bis 17, das einen zusätzlichen Virusaufreinigungsschritt umfasst.

19. Verfahren nach einem der Ansprüche 1 bis 18, das einen zusätzlichen Virusinaktivierungsschritt umfasst.

20. Verwendung eines Verfahrens nach einem der Ansprüche 1 bis 19 zur Herstellung eines Impfstoffs zur Vorbeugung gegen Grippe.

21. Verwendung eines Verfahrens nach einem der Ansprüche 1 bis 19 zur Herstellung eines Impfstoffs zur Vorbeugung gegen Humaninfluenzapandemie.

22. Verwendung eines Verfahrens nach einem der Ansprüche 1 bis 19 zur Herstellung eines Impfstoffs zur Vorbeugung gegen Humaninfluenzaepidemie.

23. Verwendung eines Verfahrens nach einem der Ansprüche 1 bis 19 zur Herstellung eines Impfstoffs zur Vorbeugung gegen Grippe bei Equidae, Suidae, Canidae, Felidae, Mustelidae und Vogelarten.

24. Verwendung von Eiern von Hühnern, die gegen Grippe geimpft wurden, zur Produktion eines Grippevirus.

25. Verwendung von Eiern von Hühnern, die gegen Grippe geimpft wurden, zur Herstellung eines Grippeimpfstoffs.

26. Verwendung von Eiern von Hühnern, die gegen Grippe geimpft wurden, nach Anspruch 24 oder 25, wobei die Eier Antikörper gegen das Hämagglutinin des Grippevirus enthalten.

27. Verwendung nach Anspruch 26, wobei die Antikörper gegen das Hämagglutinin von Grippevirus mit dem Subtyp H5, H6, H7 oder H9 gerichtet sind.

## Claims

1. Method for producing the flu virus according to which:
a. a flu vaccine is administered to hens,
b. the eggs are taken from the immunized hens,
c. the embryogenesis process is triggered,
d. the embryonated eggs are infected by inoculating a flu virus into the allantoic cavity,
e. the infected embryonated eggs are incubated under temperature and humidity conditions which allow replication of the virus, and
f. the allantoic fluid containing the virus is harvested.

2. Method according to Claim 1, according to which the vaccine protects the hens against avian flu.

3. Method according to Claim 1 or 2, according to which the flu vaccine comprises, in its composition, the haemagglutinin of a flu virus in the form of protein and/or of a gene encoding this protein.

4. Method according to one of Claims 1 to 3, according to which the composition of the flu vaccine contains an inactivated whole flu virus.

5. Method according to one of Claims 1 to 4, according to which the composition of the flu vaccine contains a product derived from a whole flu virus.

6. Method according to one of Claims 1 to 5, according to which the composition of the vaccine also contains an adjuvant.

7. Method according to one of Claims 1 to 3, according to which the composition of the flu vaccine contains an attenuated flu virus.

8. Method according to one of Claims 1 to 3, according to which the flu vaccine comprises a vector comprising a gene encoding the haemagglutinin of a flu virus.

9. Method according to Claim 8, according to which this vector is a poxvirus.

10. Method according to Claim 8 or 9, according to which the vector also comprises a gene encoding the neuraminidase of a flu virus.

11. Method according to one of Claims 8 to 10, according to which the composition of the vaccine also contains an adjuvant.

12. Method according to one of Claims 3 to 11, according to which the flu virus haemagglutinin in the form of protein and/or of a gene encoding this protein, contained in the composition of the vaccine which is administered to the hens and the haemagglutinin of the flu virus which is used to infect the allantoic cavity of the embryonated eggs from the immunized hens, have different subtypes.

13. Method according to one of Claims 3 to 11, according to which the flu virus haemagglutinin in the form of protein and/or of a gene encoding this protein, contained in the composition of the vaccine which is administered to the hens and the haemagglutinin of the flu virus which is used to infect the allantoic cavity of the embryonated eggs from the immunized hens, have the same subtype.

14. Method according to one of Claims 3 to 11, according to which the flu virus haemagglutinin in the form of protein and/or of a gene encoding this protein, contained in the composition of the vaccine which is administered to the hens and the haemagglutinin of the flu virus which is used to infect the allantoic cavity of the embryonated eggs from the immunized hens, are identical.

15. Method according to one of Claims 4, 6 or 7, according to which the flu virus contained in the composition of the vaccine which is administered to the hens is identical to the flu virus which is used to infect the allantoic cavity of the embryonated eggs from the immunized hens.

16. Method according to one of Claims 12 to 15, according to which the flu virus haemagglutinin in the form of protein and/or of a gene encoding this protein, contained in the composition of the vaccine which is administered to the hens and the haemagglutinin of the flu virus which is used to infect the allantoic cavity of the embryonated eggs from the immunized hens, have the H5, H6, H7 or H9 subtype.

17. Method according to Claim 16, according to which the flu virus haemagglutinin in the form of protein and/or of a gene encoding this protein, contained in the composition of the vaccine which is used to immunize the hens against the flu, and the haemagglutinin of the flu virus which is used to infect the allantoic cavity of the embryonated eggs from the immunized hens, have the H5 or H7 subtype.

18. Method according to one of Claims 1 to 17, comprising an additional step of purification of the virus.

19. Method according to one of Claims 1 to 18, comprising an additional step of inactivation of the virus.

20. Use of a method according to one of Claims 1 to 19, for the manufacture of a vaccine for use in preventing the flu.

21. Use of a method according to one of Claims 1 to 19, for the manufacture of a vaccine for use in preventing pandemic human flu.

22. Use of a method according to one of Claims 1 to 19, for the manufacture of a vaccine for use in preventing epidemic human flu.

23. Use of a method according to one of Claims 1 to 19, for the manufacture of a vaccine for use in preventing the flu in members of the equine family, members of the porcine family, members of the canine family, members of the feline family, mustelids and avian species.

24. Use of the eggs from hens immunized against the flu, for the production of a flu virus.

25. Use of the eggs from hens immunized against the flu, for the manufacture of a flu vaccine.

26. Use of the eggs from hens immunized against the flu according to Claim 24 or 25, according to which the eggs contain antibodies directed against the haemagglutinin of the flu virus.

27. Use according to Claim 26, according to which the antibodies are directed against the haemagglutinin of the flu virus having the H5, H6, H7 or H9 subtype.


### SEQUENCE LISTING

<110> Sanofi Pasteur
   MERIAL Limited
<120> Procédé de production du virus de la grippe
<130> PM 0706
<140> 0757884 <141> 2007-09-26
<160> 3
<170> PatentIn version 3.3
<210> 1
   <211> 564
   <212> PRT
   <213> Avian influenza virus
<220>
   <221> Séquence de la protéine HA du virus de la grippe aviaire A/chicken/Indonesia/7/03
   <222> (1)..(564)
   <223> Séquence en acide aminé codée par le gène synthétique HA modifié au niveau du site de clivage
<400> 1
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Leu Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Ser Gly Val Ser Ser Ala Cys Pro Tyr Gln Gly Lys Ser Ser Phe Phe
   145                 150                 155                 160
   Arg Asn Val Val Trp Leu Ile Lys Lys Asn Ser Ala Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Ile Arg Ser Lys Val Asn Gly Gln Ser Gly
   225                 230                 235                 240
   Arg Met Glu Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Leu Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Thr Arg Gly Leu Phe Gly Ala Ile Ala Gly Phe Ile
               340                 345                 350
   Glu Gly Gly Trp Gln Gly Met Val Asp Gly Trp Tyr Gly Tyr His His
           355                 360                 365
   Ser Asn Glu Gln Gly Ser Gly Tyr Ala Ala Asp Lys Glu Ser Thr Gln
       370                 375                 380
   Lys Ala Ile Asp Gly Val Thr Asn Lys Val Asn Ser Ile Ile Asp Lys
   385                 390                 395                 400
   Met Asn Thr Gln Phe Glu Ala Val Gly Arg Glu Phe Asn Asn Leu Glu
                   405                 410                 415
   Arg Arg Ile Glu Asn Leu Asn Lys Lys Met Glu Asp Gly Phe Leu Asp
               420                 425                 430
   Val Trp Thr Tyr Asn Ala Glu Leu Leu Val Leu Met Glu Asn Glu Arg
           435                 440                 445
   Thr Leu Asp Phe His Asp Ser Asn Val Lys Asn Leu Tyr Asp Lys Val
       450                 455                 460
   Arg Leu Gln Leu Arg Asp Asn Ala Lys Glu Leu Gly Asn Gly Cys Phe
   465                 470                 475                 480
   Glu Phe Tyr His Lys Cys Asp Asn Glu Cys Met Glu Ser Ile Arg Asn
                   485                 490                 495
   Gly Thr Tyr Asn Tyr Pro Gln Tyr Ser Glu Glu Ala Arg Leu Lys Arg
               500                 505                 510
   Glu Glu Ile Ser Gly Val Lys Leu Glu Ser Ile Gly Thr Tyr Gln Ile
           515                 520                 525
   Leu Ser Ile Tyr Ser Thr Val Ala Ser Ser Leu Ala Leu Ala Ile Met
       530                 535                 540
   Met Ala Gly Leu Ser Leu Trp Met Cys Ser Asn Gly Ser Leu Gln Cys
   545                 550                 555                 560
   Arg Ile Cys Ile
<210> 2
   <211> 7618
   <212> DNA
   <213> Artificial
<220>
   <223> plasmide donneur pJY1394.1
<400> 2
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgaat tgtaatacga ctcactatag ggcgaattgg gtgacccttt 660
   acaagaataa aagaagaaac aactgtgaaa tagtttataa atgtaattcg tatgcagaaa 720
   acgataatat attttggtat gagaaatcta aaggagacat agtttgtata gacatgcgct 780
   cttccgatga gatattcgat gcttttctaa tgtatcatat agctacaaga tatgcctatc 840
   atgatgatga tatatatcta caaatagtgt tatattattc taataatcaa aatgttatat 900
   cttatattac gaaaaataaa tacgttaagt atataagaaa taaaactaga gacgatattc 960
   ataaagtaaa aatattagct ctagaagact ttacaacgga agaaatatat tgttggatta 1020
   gtaatatata acagcgtagc tgcacggttt tgatcatttt ccaacaatat aaaccaatga 1080
   aggaggacga ctcatcaaac ataaataaca ttcacggaaa atattcagta tcagatttat 1140
   cacaagatga ttatgttatt gaatgtatag acggatcttt tgattcgatc aagtatagag 1200
   atataaaggt tataataatg aagaataacg gttacgttaa ttgtagtaaa ttatgtaaaa 1260
   tgcggaataa atacttttct agatggttgc gtctttctac ttctaaagca ttattagaca 1320
   tttacaataa taagtcagta gataatgcta ttgttaaagt ctatggtaaa ggtaagaaac 1380
   ttattataac aggattttat ctcaaacaaa atatgatacg ttatgttatt gagtggatag 1440
   gggatgattt tacaaacgat atatacaaaa tgattaattt ctataatgcg ttattcggta 1500
   acgatgaatt aaaaatagta tcctgtgaaa acactctatg cccgtttata gaacttggta 1560
   gatgctatta tggtaaaaaa tgtaagtata tacacggaga tcaatgtgat atctgtggtc 1620
   tatatatact acaccctacc gatattaacc aacgagtttc tcacaagaaa acttgtttag 1680
   tagatagaga ttctttgatt gtgtttaaaa gaagtaccag taaaaagtgt ggcatatgca 1740
   tagaagaaat aaacaaaaaa catatttccg aacagtattt tggaattctc ccaagttgta 1800
   aacatatttt ttgcctatca tgtataagac gttgggcaga tactaccaga aatacagata 1860
   ctgaaaatac gtgtcctgaa tgtagaatag tttttccttt cataataccc agtaggtatt 1920
   ggatagataa taaatatgat aaaaaaatat tatataatag atataagaaa atgattttta 1980
   caaaaatacc tataagaaca ataaaaatat aattacattt acggaaaata gctggtttta 2040
   gtttaccaac ttagagtaat tatcatattg aatctatatt gctaattagc taataaaaac 2100
   ccgggttaat taattagtca tcaggcaggg cgagaacgag actatctgct cgttaattaa 2160
   ttagagcttc tttattctat acttaaaaag tgaaaataaa tacaaaggtt cttgagggtt 2220
   gtgttaaatt gaaagcgaga aataatcata aattatttca ttatcgcgat atccgttaag 2280
   tttgtatcgt aatggagaaa atcgtgctgc tgctggccat cgtgagcctg gtgaaaagcg 2340
   atcagatctg catcggctac cacgccaaca acagcacaga gcaagtggac acaatcatgg 2400
   aaaagaacgt gaccgtgaca cacgcccagg acatcctgga aaagacacac aacgggaagc 2460
   tgtgcgatct ggatggagtg aagcctctga tcctgagaga ttgcagcgtg gccggatggc 2520
   tgctggggaa cccaatgtgc gacgaattca tcaacgtgcc cgaatggagc tacatcgtgg 2580
   agaaggccaa cccagccaac gacctgtgct acccagggaa cctgaacgac tacgaagaac 2640
   tgaaacacct gctgagcaga atcaaccact ttgagaaaat ccagatcatc cccaaaagca 2700
   gctggtccga tcacgaagcc agcagcggag tgagcagcgc ctgcccatac cagggaaagt 2760
   ccagcttttt tagaaacgtg gtgtggctga tcaaaaagaa cagcgcctac ccaacaatca 2820
   agagaagcta caacaacacc aaccaggaag atctgctggt gctgtggggg atccaccacc 2880
   ctaacgatgc cgccgagcag acaaggctgt accagaaccc aaccacctac atctccgtgg 2940
   ggacaagcac actgaaccag agactggtgc caaaaatcgc catcagatcc aaagtgaacg 3000
   ggcagagcgg aagaatggag ttcttctgga caatcctgaa acccaacgat gccatcaact 3060
   tcgagagcaa cggaaacttc atcgccccag aatacgccta caaaatcgtg aagaaagggg 3120
   acagcgccat catgaaaagc gaactggaat acggcaactg caacaccaag tgccagaccc 3180
   caatgggggc catcaacagc agcatgccat tccacaacat ccaccctctg accatcgggg 3240
   aatgccccaa atacgtgaaa agcaacagac tggtgctggc caccgggctg agaaacagcc 3300
   ctcagagaga gaccagagga ctgtttggag ccatcgccgg ctttatcgag ggaggatggc 3360
   agggaatggt ggatggctgg tacggatacc accacagcaa cgagcagggg agcggatacg 3420
   ccgccgacaa agaatccacc cagaaggcca tcgacggcgt gaccaacaaa gtgaacagca 3480
   tcatcgacaa aatgaacacc cagtttgagg ccgtgggaag ggagtttaac aacctggaaa 3540
   ggagaatcga gaacctgaac aagaagatgg aggacggatt cctggatgtg tggacctaca 3600
   acgccgaact gctggtgctg atggaaaacg agagaaccct ggactttcac gacagcaacg 3660
   tgaagaacct gtacgacaaa gtgaggctgc agctgaggga taacgccaag gagctgggca 3720
   acggctgctt cgagttctac cacaaatgcg ataacgaatg catggaaagc atcagaaacg 3780
   gaacctacaa ctacccccag tacagcgaag aagccagact gaaaagagaa gaaatctccg 3840
   gagtgaaact ggaatccatc ggaacctacc agatcctgag catctacagc acagtggcct 3900
   cctccctggc cctggccatc atgatggccg gactgagcct gtggatgtgc tccaacggaa 3960
   gcctgcagtg cagaatctgc atctgactcg agtttttatt gactagttaa tcataagata 4020
   aataatatac agcattgtaa ccatcgtcat ccgttatacg gggaataata ttaccataca 4080
   gtattattaa attttcttac gaagaatata gatcggtatt tatcgttagt ttattttaca 4140
   tttattaatt aaacatgtct actattacct gttatggaaa tgacaaattt agttatataa 4200
   tttatgataa aattaagata ataataatga aatcaaataa ttatgtaaat gctactagat 4260
   tatgtgaatt acgaggaaga aagtttacga actggaaaaa attaagtgaa tctaaaatat 4320
   tagtcgataa tgtaaaaaaa ataaatgata aaactaacca gttaaaaacg gatatgatta 4380
   tatacgttaa ggatattgat cataaaggaa gagatacttg cggttactat gtacaccaag 4440
   atctggtatc ttctatatca aattggatat ctccgttatt cgccgttaag gtaaataaaa 4500
   ttattaacta ttatatatgt aatgaatatg atatacgact tagcgaaatg gaatctgata 4560
   tgacagaagt aatagatgta gttgataaat tagtaggagg atacaatgat gaaatagcag 4620
   aaataatata tttgtttaat aaatttatag aaaaatatat tgctaacata tcgttatcaa 4680
   ctgaattatc tagtatatta aataatttta taaattttaa taaaaaatac aataacgaca 4740
   taaaagatat taaatcttta attcttgatc tgaaaaacac atctataaaa ctagataaaa 4800
   agttattcga taaagataat aatgaatcga acgatgaaaa attggaaaca gaagttgata 4860
   agctaatttt tttcatctaa atagtattat tttattgaag tacgaagttt tacgttagat 4920
   aaataataaa ggtcgatttt tattttgtta aatatcaaat atgtcattat ctgataaaga 4980
   tacaaaaaca cacggtgatt atcaaccatc taacgaacag atattacaaa aaatacgtcg 5040
   gactatggaa aacgaagctg atagcctcaa tagaagaagc attaaagaaa ttgttgtaga 5100
   tgttatgaag aattgggatc atcctctcaa cgaagaaata gataaagttc taaactggaa 5160
   aaatgataca ttaaacgatt tagatcatct aaatacagat gataatatta aggaaatcat 5220
   acaatgtctg attagagaat ttgcgtttaa aaagatcaat tctattatgt atagttatgc 5280
   tatggtaaaa ctcaattcag ataacgaaac attgaaagat aaaattaagg attattttat 5340
   agaaactatt cttaaagaca aacgtggtta taaacaaaag ccattaccct agagcggccg 5400
   ccaccgcggt ggagctccag cttttgttcc ctttagtgag ggttaatttc gagcttggcg 5460
   taatcatggt catagctgtt tcctgtgtga aattgttatc cgctcacaat tccacacaac 5520
   atacgagccg gaagcataaa gtgtaaagcc tggggtgcct aatgagtgag ctaactcaca 5580
   ttaattgcgt tgcgctcact gcccgctttc cagtcgggaa acctgtcgtg ccagctgcat 5640
   taatgaatcg gccaacgcgc ggggagaggc ggtttgcgta ttgggcgctc ttccgcttcc 5700
   tcgctcactg actcgctgcg ctcggtcgtt cggctgcggc gagcggtatc agctcactca 5760
   aaggcggtaa tacggttatc cacagaatca ggggataacg caggaaagaa catgtgagca 5820
   aaaggccagc aaaaggccag gaaccgtaaa aaggccgcgt tgctggcgtt tttccatagg 5880
   ctccgccccc ctgacgagca tcacaaaaat cgacgctcaa gtcagaggtg gcgaaacccg 5940
   acaggactat aaagatacca ggcgtttccc cctggaagct ccctcgtgcg ctctcctgtt 6000
   ccgaccctgc cgcttaccgg atacctgtcc gcctttctcc cttcgggaag cgtggcgctt 6060
   tctcatagct cacgctgtag gtatctcagt tcggtgtagg tcgttcgctc caagctgggc 6120
   tgtgtgcacg aaccccccgt tcagcccgac cgctgcgcct tatccggtaa ctatcgtctt 6180
   gagtccaacc cggtaagaca cgacttatcg ccactggcag cagccactgg taacaggatt 6240
   agcagagcga ggtatgtagg cggtgctaca gagttcttga agtggtggcc taactacggc 6300
   tacactagaa ggacagtatt tggtatctgc gctctgctga agccagttac cttcggaaaa 6360
   agagttggta gctcttgatc cggcaaacaa accaccgctg gtagcggtgg tttttttgtt 6420
   tgcaagcagc agattacgcg cagaaaaaaa ggatctcaag aagatccttt gatcttttct 6480
   acggggtctg acgctcagtg gaacgaaaac tcacgttaag ggattttggt catgagatta 6540
   tcaaaaagga tcttcaccta gatcctttta aattaaaaat gaagttttaa atcaatctaa 6600
   agtatatatg agtaaacttg gtctgacagt taccaatgct taatcagtga ggcacctatc 6660
   tcagcgatct gtctatttcg ttcatccata gttgcctgac tccccgtcgt gtagataact 6720
   acgatacggg agggcttacc atctggcccc agtgctgcaa tgataccgcg agacccacgc 6780
   tcaccggctc cagatttatc agcaataaac cagccagccg gaagggccga gcgcagaagt 6840
   ggtcctgcaa ctttatccgc ctccatccag tctattaatt gttgccggga agctagagta 6900
   agtagttcgc cagttaatag tttgcgcaac gttgttgcca ttgctacagg catcgtggtg 6960
   tcacgctcgt cgtttggtat ggcttcattc agctccggtt cccaacgatc aaggcgagtt 7020
   acatgatccc ccatgttgtg caaaaaagcg gttagctcct tcggtcctcc gatcgttgtc 7080
   agaagtaagt tggccgcagt gttatcactc atggttatgg cagcactgca taattctctt 7140
   actgtcatgc catccgtaag atgcttttct gtgactggtg agtactcaac caagtcattc 7200
   tgagaatagt gtatgcggcg accgagttgc tcttgcccgg cgtcaatacg ggataatacc 7260
   gcgccacata gcagaacttt aaaagtgctc atcattggaa aacgttcttc ggggcgaaaa 7320
   ctctcaagga tcttaccgct gttgagatcc agttcgatgt aacccactcg tgcacccaac   7380
   tgatcttcag catcttttac tttcaccagc gtttctgggt gagcaaaaac aggaaggcaa   7440
   aatgccgcaa aaaagggaat aagggcgaca cggaaatgtt gaatactcat actcttcctt   7500
   tttcaatatt attgaagcat ttatcagggt tattgtctca tgagcggata catatttgaa   7560
   tgtatttaga aaaataaaca aataggggtt ccgcgcacat ttccccgaaa agtgccac     7618
<210> 3
   <211> 4885
   <212> DNA
   <213> Artificial
<220>
   <223> séquence nucléotidique de l'insert du plasmide pJY1394.1
<220>
   <221> séquence partielle du plasmide de clonage comprenant la séquence du primer M13F
   <222> (1)..(53)
<220>
   <221> séquence du " bras gauche " flanquant le locus d'insertion F8 dans le génome du vecteur TROVAC fowlpox.
   <222> (54)..(1483)
<220>
   <221> séquence de liaison entre le bras gauche et le promoteur H6
   <222> (1484)..(1568)
<220>
   <221> séquence du promoteur H6 du virus de la vaccine
   <222> (1569)..(1692)
<220>
   <221> séquence du gène synthétique modifié HA de la souche A/chicken/Indonesia/7/03
   <222> (1693)..(3387)
<220>
   <221> séquence de liaison entre le gène HA et le bras droit
   <222> (3388)..(3414)
   <223> séquence comprenant une séquence TTTTTAT d'arrêt de transcription des gènes précoces du fowlpox
<220>
   <221> séquence du " bras droit " flanquant le locus d'insertion F8 dans le génome du vecteur TROVAC fowlpox
   <222> (3415)..(4790)
<220>
   <221> séquence partielle du plasmide de clonage comprenant la séquence du primer M13R
   <222> (4791)..(4885)
<400> 3
   gtaaaacgac ggccagtgaa ttgtaatacg actcactata gggcgaattg ggtgaccctt 60
   tacaagaata aaagaagaaa caactgtgaa atagtttata aatgtaattc gtatgcagaa 120
   aacgataata tattttggta tgagaaatct aaaggagaca tagtttgtat agacatgcgc 180
   tcttccgatg agatattcga tgcttttcta atgtatcata tagctacaag atatgcctat 240
   catgatgatg atatatatct acaaatagtg ttatattatt ctaataatca aaatgttata 300
   tcttatatta cgaaaaataa atacgttaag tatataagaa ataaaactag agacgatatt 360
   cataaagtaa aaatattagc tctagaagac tttacaacgg aagaaatata ttgttggatt 420
   agtaatatat aacagcgtag ctgcacggtt ttgatcattt tccaacaata taaaccaatg 480
   aaggaggacg actcatcaaa cataaataac attcacggaa aatattcagt atcagattta 540
   tcacaagatg attatgttat tgaatgtata gacggatctt ttgattcgat caagtataga 600
   gatataaagg ttataataat gaagaataac ggttacgtta attgtagtaa attatgtaaa 660
   atgcggaata aatacttttc tagatggttg cgtctttcta cttctaaagc attattagac 720
   atttacaata ataagtcagt agataatgct attgttaaag tctatggtaa aggtaagaaa 780
   cttattataa caggatttta tctcaaacaa aatatgatac gttatgttat tgagtggata 840
   ggggatgatt ttacaaacga tatatacaaa atgattaatt tctataatgc gttattcggt 900
   aacgatgaat taaaaatagt atcctgtgaa aacactctat gcccgtttat agaacttggt 960
   agatgctatt atggtaaaaa atgtaagtat atacacggag atcaatgtga tatctgtggt 1020
   ctatatatac tacaccctac cgatattaac caacgagttt ctcacaagaa aacttgttta 1080
   gtagatagag attctttgat tgtgtttaaa agaagtacca gtaaaaagtg tggcatatgc 1140
   atagaagaaa taaacaaaaa acatatttcc gaacagtatt ttggaattct cccaagttgt 1200
   aaacatattt tttgcctatc atgtataaga cgttgggcag atactaccag aaatacagat 1260
   actgaaaata cgtgtcctga atgtagaata gtttttcctt tcataatacc cagtaggtat 1320
   tggatagata ataaatatga taaaaaaata ttatataata gatataagaa aatgattttt 1380
   acaaaaatac ctataagaac aataaaaata taattacatt tacggaaaat agctggtttt 1440
   agtttaccaa cttagagtaa ttatcatatt gaatctatat tgctaattag ctaataaaaa 1500
   cccgggttaa ttaattagtc atcaggcagg gcgagaacga gactatctgc tcgttaatta 1560
   attagagctt ctttattcta tacttaaaaa gtgaaaataa atacaaaggt tcttgagggt 1620
   tgtgttaaat tgaaagcgag aaataatcat aaattatttc attatcgcga tatccgttaa 1680
   gtttgtatcg taatggagaa aatcgtgctg ctgctggcca tcgtgagcct ggtgaaaagc 1740
   gatcagatct gcatcggcta ccacgccaac aacagcacag agcaagtgga cacaatcatg 1800
   gaaaagaacg tgaccgtgac acacgcccag gacatcctgg aaaagacaca caacgggaag 1860
   ctgtgcgatc tggatggagt gaagcctctg atcctgagag attgcagcgt ggccggatgg 1920
   ctgctgggga acccaatgtg cgacgaattc atcaacgtgc ccgaatggag ctacatcgtg 1980
   gagaaggcca acccagccaa cgacctgtgc tacccaggga acctgaacga ctacgaagaa 2040
   ctgaaacacc tgctgagcag aatcaaccac tttgagaaaa tccagatcat ccccaaaagc 2100
   agctggtccg atcacgaagc cagcagcgga gtgagcagcg cctgcccata ccagggaaag 2160
   tccagctttt ttagaaacgt ggtgtggctg atcaaaaaga acagcgccta cccaacaatc 2220
   aagagaagct acaacaacac caaccaggaa gatctgctgg tgctgtgggg gatccaccac 2280
   cctaacgatg ccgccgagca gacaaggctg taccagaacc caaccaccta catctccgtg 2340
   gggacaagca cactgaacca gagactggtg ccaaaaatcg ccatcagatc caaagtgaac 2400
   gggcagagcg gaagaatgga gttcttctgg acaatcctga aacccaacga tgccatcaac 2460
   ttcgagagca acggaaactt catcgcccca gaatacgcct acaaaatcgt gaagaaaggg 2520
   gacagcgcca tcatgaaaag cgaactggaa tacggcaact gcaacaccaa gtgccagacc 2580
   ccaatggggg ccatcaacag cagcatgcca ttccacaaca tccaccctct gaccatcggg 2640
   gaatgcccca aatacgtgaa aagcaacaga ctggtgctgg ccaccgggct gagaaacagc 2700
   cctcagagag agaccagagg actgtttgga gccatcgccg gctttatcga gggaggatgg 2760
   cagggaatgg tggatggctg gtacggatac caccacagca acgagcaggg gagcggatac 2820
   gccgccgaca aagaatccac ccagaaggcc atcgacggcg tgaccaacaa agtgaacagc 2880
   atcatcgaca aaatgaacac ccagtttgag gccgtgggaa gggagtttaa caacctggaa 2940
   aggagaatcg agaacctgaa caagaagatg gaggacggat tcctggatgt gtggacctac 3000
   aacgccgaac tgctggtgct gatggaaaac gagagaaccc tggactttca cgacagcaac 3060
   gtgaagaacc tgtacgacaa agtgaggctg cagctgaggg ataacgccaa ggagctgggc 3120
   aacggctgct tcgagttcta ccacaaatgc gataacgaat gcatggaaag catcagaaac 3180
   ggaacctaca actaccccca gtacagcgaa gaagccagac tgaaaagaga agaaatctcc 3240
   ggagtgaaac tggaatccat cggaacctac cagatcctga gcatctacag cacagtggcc 3300
   tcctccctgg ccctggccat catgatggcc ggactgagcc tgtggatgtg ctccaacgga 3360
   agcctgcagt gcagaatctg catctgactc gagtttttat tgactagtta atcataagat 3420
   aaataatata cagcattgta accatcgtca tccgttatac ggggaataat attaccatac 3480
   agtattatta aattttctta cgaagaatat agatcggtat ttatcgttag tttattttac 3540
   atttattaat taaacatgtc tactattacc tgttatggaa atgacaaatt tagttatata 3600
   atttatgata aaattaagat aataataatg aaatcaaata attatgtaaa tgctactaga 3660
   ttatgtgaat tacgaggaag aaagtttacg aactggaaaa aattaagtga atctaaaata   3720
   ttagtcgata atgtaaaaaa aataaatgat aaaactaacc agttaaaaac ggatatgatt   3780
   atatacgtta aggatattga tcataaagga agagatactt gcggttacta tgtacaccaa   3840
   gatctggtat cttctatatc aaattggata tctccgttat tcgccgttaa ggtaaataaa   3900
   attattaact attatatatg taatgaatat gatatacgac ttagcgaaat ggaatctgat   3960
   atgacagaag taatagatgt agttgataaa ttagtaggag gatacaatga tgaaatagca   4020
   gaaataatat atttgtttaa taaatttata gaaaaatata ttgctaacat atcgttatca   4080
   actgaattat ctagtatatt aaataatttt ataaatttta ataaaaaata caataacgac   4140
   ataaaagata ttaaatcttt aattcttgat ctgaaaaaca catctataaa actagataaa   4200
   aagttattcg ataaagataa taatgaatcg aacgatgaaa aattggaaac agaagttgat   4260
   aagctaattt ttttcatcta aatagtatta ttttattgaa gtacgaagtt ttacgttaga   4320
   taaataataa aggtcgattt ttattttgtt aaatatcaaa tatgtcatta tctgataaag   4380
   atacaaaaac acacggtgat tatcaaccat ctaacgaaca gatattacaa aaaatacgtc   4440
   ggactatgga aaacgaagct gatagcctca atagaagaag cattaaagaa attgttgtag   4500
   atgttatgaa gaattgggat catcctctca acgaagaaat agataaagtt ctaaactgga   4560
   aaaatgatac attaaacgat ttagatcatc taaatacaga tgataatatt aaggaaatca   4620
   tacaatgtct gattagagaa tttgcgttta aaaagatcaa ttctattatg tatagttatg   4680
   ctatggtaaa actcaattca gataacgaaa cattgaaaga taaaattaag gattatttta   4740
   tagaaactat tcttaaagac aaacgtggtt ataaacaaaa gccattaccc tagagcggcc   4800
   gccaccgcgg tggagctcca gcttttgttc cctttagtga gggttaattt cgagcttggc   4860
   gtaatcatgg tcatagctgt ttcct                                         4885